# EUROPEAN PATENT SPECIFICATION

(11) **EP 0 906 300 B1**
(45) Date of publication and mention of the grant of the patent: **16.01.2002**
(21) Application number: 97927840.5
(22) Date of filing: 27.05.1997
(51) Int. Cl.: C07D 307/58, C07D 307/60

(54) **PROCESS FOR MAKING PHENYL HETEROCYCLES USEFUL AS COX-2 INHIBITORS**
VERFAHREN ZUR HERSTELLUNG VON PHENYLHETEROCYCLEN DIE ALS COX-2 INHIBITOREN VERWENDET WERDEN KÖNNEN
PROCEDE POUR LA FABRICATION D'HETEROCYCLES DE PHENYLE UTILES COMME INHIBITEURS DE LA CYCLO-OXYGENASE-2 (COX-2)

(30) Priority: 31.05.1996 US 18644 P; 21.06.1996 GB 9613110; 09.10.1996 US 28108 P; 09.10.1996 US 28109 P; 01.11.1996 GB 9622831; 01.11.1996 GB 9622816
(43) Date of publication of application: 07.04.1999
(73) Proprietor: MERCK & CO., INC., Rahway, New Jersey 07065 (US)
(72) Inventor: ROSSEN, Kai, Rahway, NJ 07065 (US); VOLANTE, Ralph, P., Rahway, NJ 07065 (US); HO, Guo-Jie, Rahway, NJ 07065 (US); FARR, Roger, N., Rahway, NJ 07065 (US); MATHRE, David, J., Rahway, NJ 07065 (US)
(74) Representative: Horgan, James Michael Frederic
(86) International application number: US9709193
(87) International publication number: WO9745420

(56) References cited:
- EP-A- 0 754 687
- WO-A-94/15932
- WO-A-94/26731
- WO-A-95/00501
- WO-A-96/08482
- WO-A-96/19469

## Description

### BACKGROUND OF THE INVENTION

This invention concerns a process for making certain antiinflammatory compounds. In particular, the application concerns a process for making compounds of formulas I and Ia as disclosed hereinunder, which compounds are potent cyclooxygenase-2 inhibitors.

Non-steroidal, antiinflammatory drugs exert most of their antiinflammatory, analgesic and antipyretic activity and inhibit hormone-induced uterine contractions and certain types of cancer growth through inhibition of prostaglandin G/H synthase, also known as cyclooxygenase. Up until recently, only one form of cyclooxygenase had been characterized, this corresponding to cyclooxygenase-1 or the constitutive enzyme, as originally identified in bovine seminal vesicles. Recently the gene for a second inducible form of cyclooxygenase (cyclooxygenase-2) has been cloned, sequenced and characterized from chicken, murine and human sources. This enzyme is distinct from the cyclooxygenase-1 which has now also been cloned, sequenced and characterized from sheep, murine and human sources. The second form of cyclooxygenase, cyclooxygenase-2, is rapidly and readily inducible by a number of agents including mitogens, endotoxin, hormones, cytokines and growth factors. As prostaglandins have both physiological and pathological roles, we have concluded that the constitutive enzyme, cyclooxygenase-1, is responsible, in large part, for endogenous basal release of prostaglandins and hence is important in their physiological functions such as the maintenance of gastrointestinal integrity and renal blood flow. In contrast, we have concluded that the inducible form, cyclooxygenase-2, is mainly responsible for the pathological effects of prostaglandins where rapid induction of the enzyme would occur in response to such agents as inflammatory agents, hormones, growth factors, and cytokines. Thus, a selective inhibitor of cyclooxygenase-2 will have similar antiinflammatory, antipyretic and analgesic properties to a conventional non-steroidal antiinflammatory drug, and in addition would inhibit hormone-induced uterine contractions and have potential anti-cancer effects, but will have a diminished ability to induce some of the mechanism-based side effects. In particular, such a compound should have a reduced potential for gastrointestinal toxicity, a reduced potential for renal side effects, a reduced effect on bleeding times and possibly a lessened ability to induce asthma attacks in aspirin-sensitive asthmatic subjects. WO 95/00501 discloses multi-step methods for the preparation of phenyl heterocycles for the use as cyclooxygenase -2 inhibitors.

WO 94/ 15932 published July 21, 1994 discloses a multistep method of making bi-aryl furans via bi-aryl lactones, which method utilizes a keto-ester internal cyclization to the lactone. We have found that a significant amount of undesired by-products are produced by use of the disclosed process scheme, due to the external cyclization reactions which compete with the desired internal cyclization. While these by-products can be removed by suitable separation and purification techniques, we have sought to identify alternative processes to obviate the difficulties.

### SUMMARY OF THE INVENTION

The invention encompasses a process for making compounds of Formula I and Formula Ia useful in the treatment of inflammation and other cyclooxygenase-2 mediated diseases.

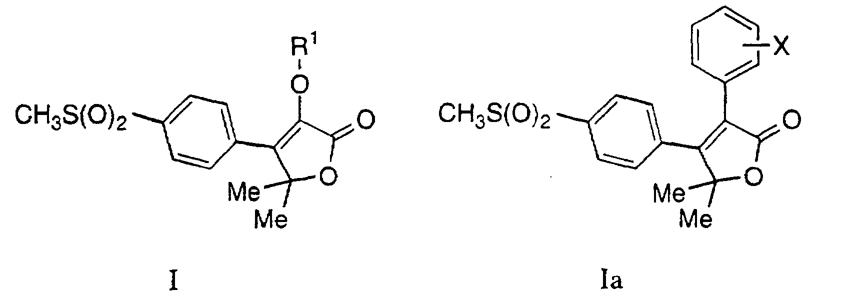

### DETAILED DESCRIPTION OF THE INVENTION

In one aspect the invention encompasses a process for making compounds of Formula I useful in the treatment of inflammation and other cyclooxygenase-2 mediated diseases 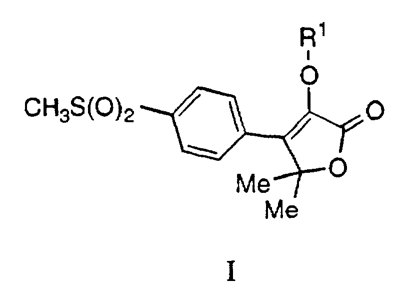
R¹ is selected from the group consisting of
   (a) linear or branched C₁₋₆alkyl,
   (b) mono-, di- or tri-substituted phenyl or naphthyl wherein the substituents are selected from the group consisting of
      (1) hydrogen,
      (2) halo,
      (3) C₁₋₃alkoxy,
      (4) CN,
      (5) C₁₋₃ fluoroalkyl
      (6) C₁₋₃ alkyl,
      (7) -CO₂H,
   the process comprising:
   (a) reacting thioanisole 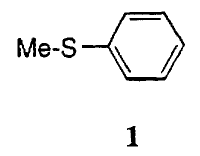 in a non-reactive solvent and in the presence of a Lewis Acid, with isobutyryl chloride 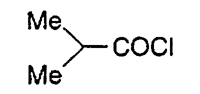 to yield compound **2** 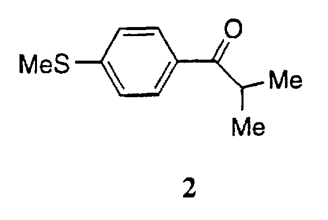
      For purposes of this specification non-reactive solvents include halocarbon and polyhalocarbon solvents such as mono or di-halo C₁₋₄ alkyl including dichloromethane; aromatic solvents such as nitrobenzene, or halogenated aromatics and C₆₋₁₀ linear, branched or cyclic hydrocarbon solvent including hexane, cyclohexane or methylcyclohexane or CS₂. For this step, the non-reactive solvents are preferably cyclohexane or ortho dichlorobenzene. Suitable Lewis Acids include but are not limited to AlCl₃, FeCl₃, TiCl₄ and SnCl₄.
      The molar ratio of thioanisole compound **1** to isobutyryl chloride may typically be varied from 1:1.5 to 1.5:1; preferably 1:1 to 1.5. Excess isobutyl chloride is typically used. Similarly, the molar ratio of compound of thioanisole compound **1** to Lewis Acid may typically be varied from 1:1.5 to 1.5:1. Preferably the molar ratio of thioanisole compound **1** to Lewis Acid is 1:1 to 1.5. The reaction step may conveniently be conducted at a temperature range of 0 to 25°C; preferably 5 to 15°C and is allowed to proceed until substantially complete in from 30 min. to 4 hours; typically 1 to 2 hours.
      The reaction is preferably conducted is the absence of moisture, preferable under nitrogen.
(b) brominating reacting a compound **2**
   with brominating agent in a non-reactive solvent (as defined above), in the presence of a second solvent, such as ethyl acetate to yield compound **3** 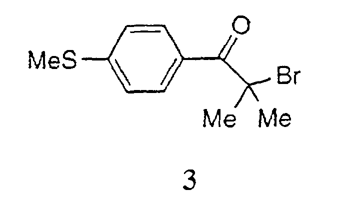
   For purposes of this specification, brominating agents are intended to include Br₂, N-bromosuccinimide and dibromo dimethyl hydantoin. The bromine may be generated in situ. The second solvent is intended to include esters such as ethyl acetate, isopropyl acetate and t-butyl acetate and etheral solvents such as etheral solvents such as diethyl ether di-n-butyl and diisopropyl ethers, cyclic ethers such as tetrahydropyran, and tetrahydrofuran. Typically, molar ratio of compound 2 to brominating agent is approximately 1:1. Most often excess brominating agent is used. The reaction step may be conducted at a temperature range of 0 to 50°C; preferably 5 to 20°C, and is allowed to proceed until substantially complete in from 30 minutes to 2 hours; typically 45 to 90 minutes.
   In an alternative aspect, compound 2 may be chlorinated by the same procedure, rather than brominated. For purposes of this specification the chlorinating agents are intended to include Cl₂, N-chlorosuccinimide and dichloro dimethyl hydantoin.
(c) oxidizing compound **3** in a non-reactive solvent (as defined above), with an oxidizing agent, optionally in the presence of a suitable catalyst to yield compound **4** 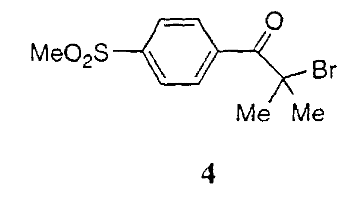
   The oxidation may be accomplished by a number of means available in the art. See, for example Can. J. Chem. 59, 720 (1981), Can. J. Chem. 60, 618 (1982), J. Chem. Soc. (C) 1969, 233, J. Org. Chem., 28, 1140 (1963), Org. Prep. Proceed. Int, 13, 137 (1981), J. Org. Chem., 50, 1544, (1985), Chem. Ber., 119, 269, (1986), and Synthesis, 1015, 1987. We have found catalysed oxidation with hydrogen peroxide to be surprisingly superior in that undesired side-reaction oxidations are minimized and environmental impact and removal of side products are good, as water is the by product.
   Suitable catalysts include sodium tungstate di-hydrate and tungstic acid.
   Typically the molar ratio of compound **3** to oxidizing agent should be approximately 1 to 2 :4, that is excess oxidizing agent is preferred. The reaction step may be conducted at a temperature range of 0 to 70 or 90°C; preferably 10 to 65 or 75°C and is allowed to proceed until substantially complete in from 1 to 5 hours; typically 2 to 4 hours.
(d) reacting compound **4** in an alkanol solvent with compound **5** 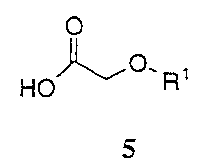 in the presence of a suitable base to yield a compound of formula **6** 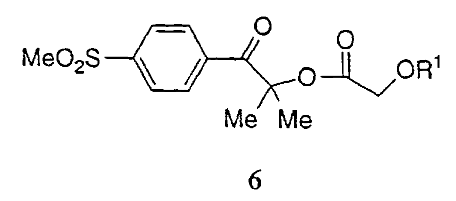
   For purposes of this specification, the alkanol solvent includes, but is not limited to ethyl alcohol. For purposes of this specification, the suitable base is intended to include diisopropylethyl amine (DIEA). Typically the molar ratio of compound **4** to compound **5** may conviently be varied from 1.5:1 to 1:1.5; prefereably 1:1 to 1.2. Excess compound **5** is typically used. The ratio of compound **4** to suitable base is typically 1: 1 to 2; preferably about 1:1.8. The reaction step a may be conducted at a temperature range of 0 to 80°C; preferably 10 to 70°C, and is allowed to proceed until substantially complete in from 2 to 20 hours; typically 8 to 16 hours.
   Isopropoxyacetic acid was prepared by addition of sodium chloroacetate to a solution of sodium isopropoxide in isopropanol, generated by reaction of sodium hydroxide and isopropanol. The reaction is typically complete after reflux for 4-5 h. The reaction is quenched by addition of water and isopropanol is removed under vacuum. The aqueous solution is acidified and saturated with sodium chloride and isopropoxyacetic acid is extracted into methyl t-butyl ether. Typically, the reaction yield is only moderate (∼75 %) due to hydrolysis. Regarding the preparation of Isopropoxyacetic acid, see also J. Chem. Soc.(c) 1969, 2698; J. Am. Chem. Soc. 1949, 71, 3372; and J. Chem Soc. Perkin. Trans. I 1983, 2479.
   The penultimate ester **6** is formed by reaction of the bromosulfone and isopropoxyacetic acid, preferably in ethanol using DIPEA as the base. Side-products include the alcohol, olefin, and keto-alcohol shown immediately below, all of which can be effectively removed by crystallization of the ester from ethanol and the isolated yield is 70-78 %. 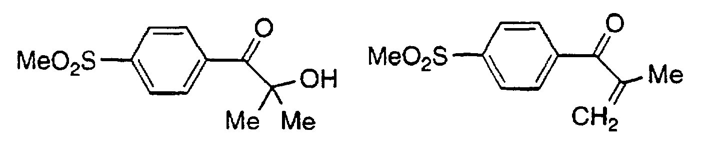 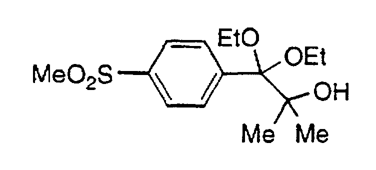
(e) reacting compound 6 in an aprotic solvent with a strong base to yield a cyclized compound of formula 7. 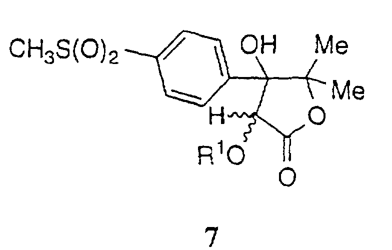 which after dehydration in the presence of a water scavenger yields a compound of formula I.

With regard to the cyclization, a strong base is required to prevent a cessation of the reaction after formation of the ester (**6**).
Thus, for purposes of this specification the strong base shall be defined to include 1,8-Diazabicyclo[5.4.0]undec-7-ene (DBU), 1,4-diazabicyclo[2.2.2]octane (DABCO) and 1,5-diazabicyclo[4.3.0]non-5-ene (DBN). For purposes of this specification the water scavenger shall be defined to include Esters of trifluoroacetic acid, such as isopropyl trifluoroacetate, esters of trichloroacetic acid and esters of alkyl or aryl sulfonic acid. Aprotic solvents shall be defined to include acetonitrile, N,N-dimethylformamide, methyl sulfoxide, propionitrile and nitromethane. Dehydration is accomplished by heating (refluxing).
The molar ratio of ester to strong base typically about 1:1 to 1: 2, with 1:1.5 preferred. The molar ratio of ester to water scavenger is typically 1:1 to 1:2, with 1:1.2 preferred. The reaction is allowed to proceed at 0 to 25° until substantially complete in 1 to 14 hours.

Bases such as potassium bis(trimethylsilyl)amide or Lithium diisopropylamide (LDA) cause the cleavage of the ester, possibly through ketene formation and are therefore less preferred. A significant amount of the above mentioned alcohol side product was formed together with several unidentified side-products, which were likely derived from the ketene. Cyclization under acidic conditions fail since ester cleavage is consistantly observed as the major reaction.

Ester **6** undergoes facile transesterification when the reaction is run in alcoholic solvents. Alcohol is formed in > 60% in IPA and exclusively in EtOH. The reaction thus has to be carried out in aprotic solvents. Even in MeCN, ∼40% of alcohol was formed at the end of reaction. The ester side product is rapidly hydrolyzed by the 1 equiv of water generated during the reacton. Therefore, an efficient water scavenger is important. Esters of trifluoroacetic acid were chosen because they would hydrolyze readily in the presence of DBU. Thus, addition of 1.2 equiv of ethyl trifluoroacetate reduced the formation of alcohol to ∼5%. The reaction is advantageously carried out in a mixture of 1.2 equiv of isopropyl trifluoroacetate and 1.5 equiv of DBU (1 equiv needed to neutralize TFA) in acetonitrile at reflux. The reaction is typically complete in 14 h and the product crystallizes upon addition of water after partial removal of MeCN. The isolated yield is 94% (98% assay yield) with purity of >99 A% at 210 nm. The overall yield for esterification and cyclization is 65%.

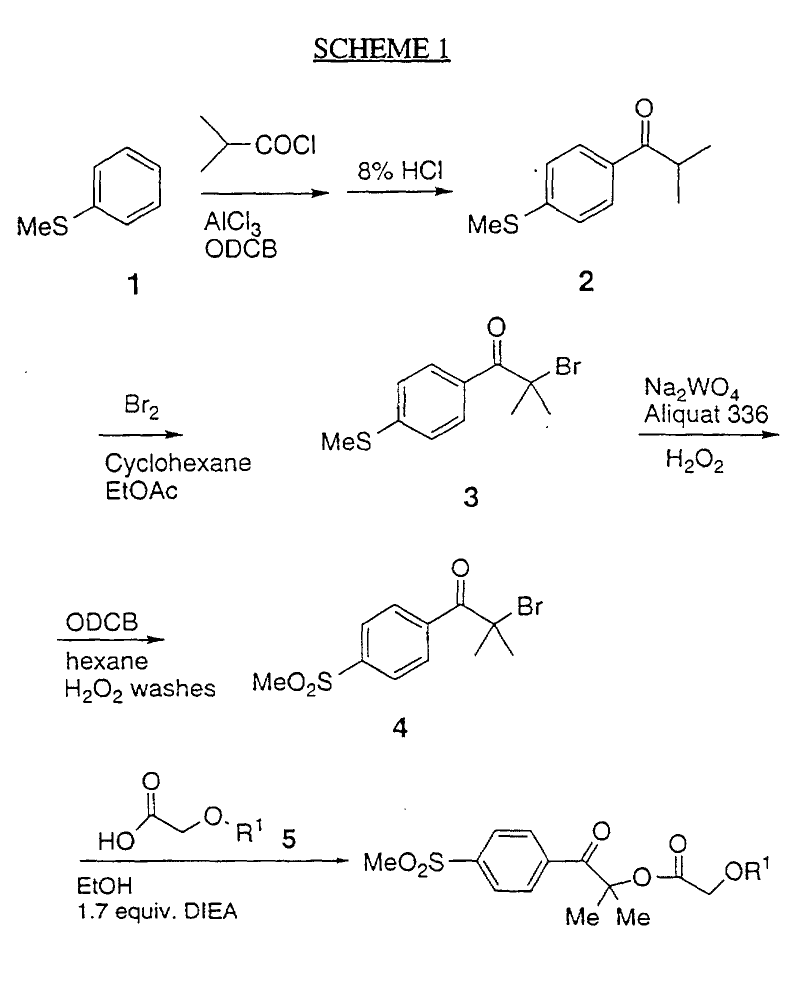

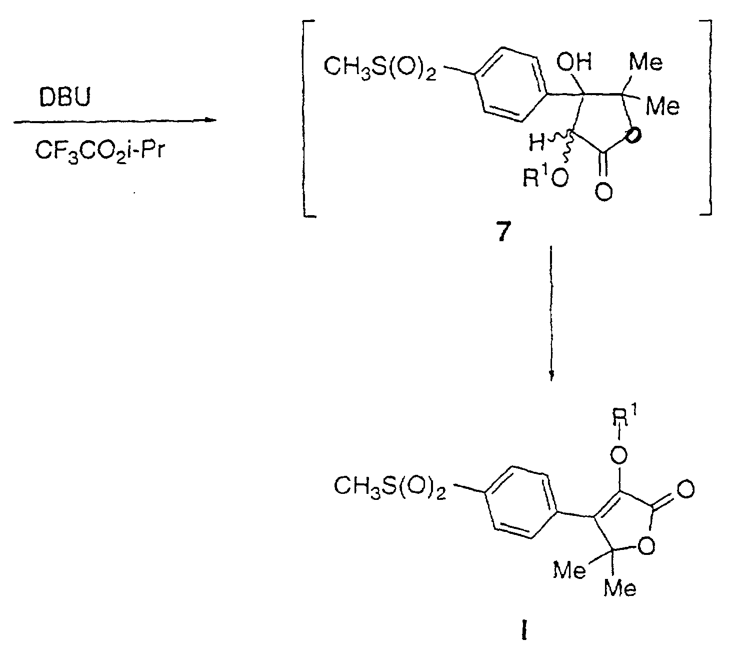

Scheme 1. Compounds of formula I are prepared in a 6 step process -- 3 steps starting from sodium chloroacetate, isopropanol, and the bromosulfone intermediate. Reaction of sodium chloroacetate with sodium isopropoxide gives isopropoxyacetic acid, which is coupled with the bromosulfone to form the ester. Cyclization and dehydration of the ester to form compounds of formula I is effected by a strong base in the presence of a water scavenger.

In an alternative procedure, compound 4 may be prepared as shown in Scheme 2 and Example 2.

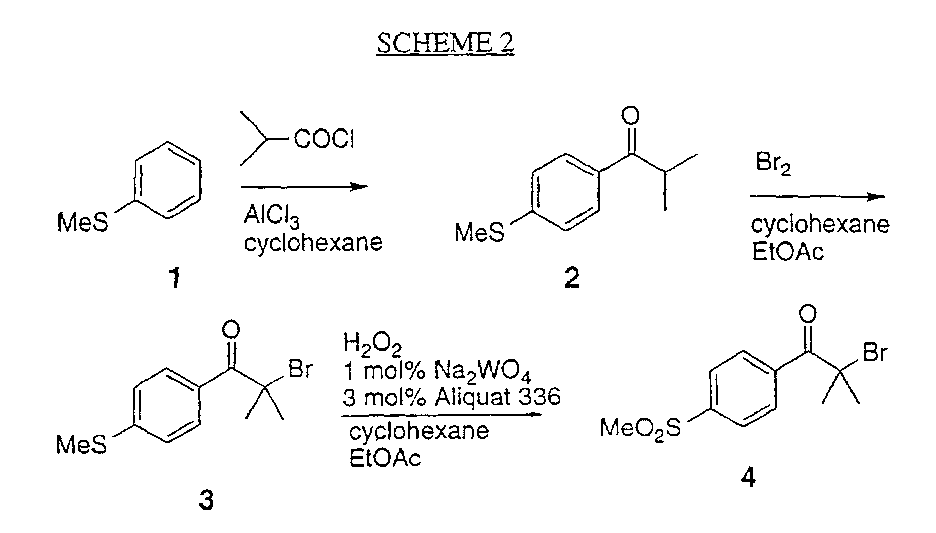

In a second aspect the invention encompasses a process for making compounds of Formula Ia useful in the treatment of inflammation and other cyclooxygenase-2 mediated diseases 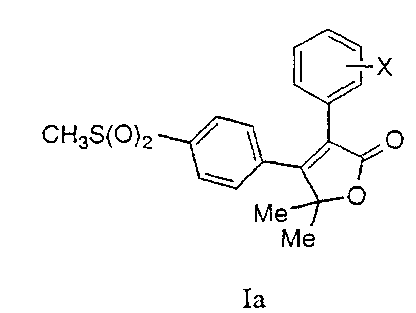
X is fluoro or chloro;
the process comprising:
(a) reacting thioanisole 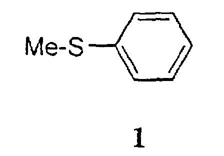 in a non-reactive solvent and in the presence of a Lewis Acid, with isobutyryl chloride 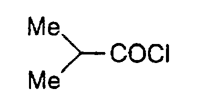 to yield compound **2** 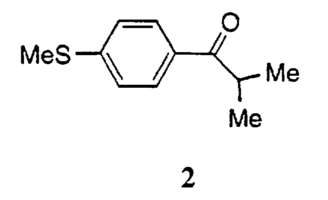
   In an alternative embodiment, there may be more than one group "X" on the phenyl and X may be defined to include H, F and Cl.
   For purposes of this specification non-reactive solvents include halocarbon and polyhalocarbon solvents such as mono or di-halo C₁₋₄ alkyl including dichloromethane; aromatic solvents such as nitrobenzene, or halogenated aromatics and C₆₋₁₀ linear, branched or cyclic hydrocarbon solvent including hexane, cyclohexane or methylcyclohexane or CS₂. For this step, the non-reactive solvents are preferably cyclohexane or ortho dichlorobenzene. Suitable Lewis Acids include but are not limited to AlCl₃, FeCl₃, TiCl₄ and SnCl₄.
   The molar ratio of thioanisole compound **1** to isobutyryl chloride may typically be varied from 1:1.5 to 1.5:1; preferably 1:1 to 1.5. Excess isobutyl chloride is typically used. Similarly, the molar ratio of compound of thioanisole compound **1** to Lewis Acid may typically be varied from 1:1.5 to 1.5:1. Preferably the molar ratio of thioanisole compound **1** to Lewis Acid is 1:1 to 1.5. The reaction step may conveniently be conducted at a temperature range of 0 to 25°C; preferably 5 to 15°C and is allowed to proceed until substantially complete in from 30 min. to 4 hours; typically 1 to 2 hours.
   The reaction is preferably conducted is the absence of moisture, preferable under nitrogen.
(b) brominating reacting a compound **2** with brominating agent in a non-reactive solvent (as defined above), in the presence of a second solvent, such as ethyl acetate to yield compound **3** 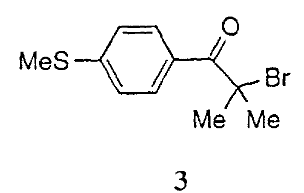
   For purposes of this specification, brominating agent are intended to include Br₂, N-bromosuccinimide and dibromo dimethyl hydantoin. The bromine may be generated in situ. The second solvent is intended to include esters such as ethyl acetate, isopropyl acetate and t-butyl acetate and etheral solvents such as diethyl ether di-n-butyl and diisopropyl ethers, cyclic ethers such as tetrahydropyran, and tetrahydrofuran. Typically, molar ratio of compound **2** to brominating agent is approximately 1:1. Most often excess brominating agent is used. The reaction step may be conducted at a temperature range of 0 to 50°C; preferably 5 to 20°C, and is allowed to proceed until substantially complete in from 30 minutes to 2 hours; typically 45 to 90 minutes.
   In an alternative aspect, compound **2** may be chlorinated by the same procedure, rather than brominated. For purposes of this specification the chlorinating agents are intended to include Cl₂, N-chlorosuccinimide and dichloro dimethyl hydantoin.
(c) oxidizing compound **3** in a non-reactive solvent (as defined above), with an oxidizing agent, optionally in the presence of a suitable catalyst to yield compound **4** 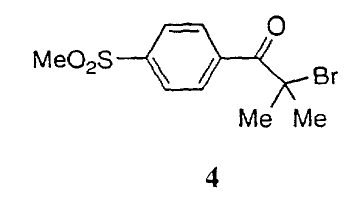
   The oxidation may be accomplished by a number of means available in the art. See, for example Can. J. Chem. 59, 720 (1981), Can. J. Chem. 60, 618 (1982), J. Chem. Soc. (C) 1969, 233, J. Org. Chem., 28, 1140 (1963), Org. Prep. Proceed. Int, 13, 137 (1981), J. Org. Chem., 50, 1544, (1985), Chem. Ber., 119, 269, (1986), and Synthesis, 1015, 1987. We have found catalysed oxidation with hydrogen peroxide to be surprisingly superior in that undesired side-reaction oxidations are minimized and environmental impact and removal of side products are good, as water is the by product.
   Suitable catalysts include sodium tungstate di-hydrate and tungstic acid.
   Typically the molar ratio of compound **3** to oxidizing agent should be approximately 1 to 2 :4, that is excess oxidizing agent is preferred. The reaction step may be conducted at a temperature range of 0 to 70 or 90°C; preferably 10 to 65 or 75°C and is allowed to proceed until substantially complete in from 1 to 5 hours; typically 2 to 4 hours.
(d) reacting compound **4** in an alkanol solvent with compound **5a** 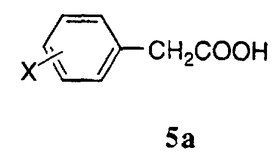 wherein X is F or Cl;
   in the presence of a suitable base to yield a compound of Formula Ia

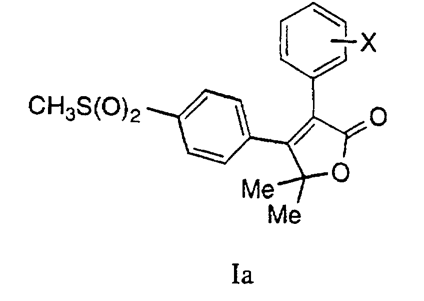

For purposes of this specification, the alkanol solvent includes, but is not limited to ethyl alcohol. For purposes of this specification, the suitable base is intended to include diisopropylethyl amine. Typically the molar ratio of compound **4** to compound **5a** may conviently be varied from 1.5:1 to 1:1.5; prefereably 1:1 to 1.2. Excess compound **5a** is typically used. The ratio of compound **4** to suitable base is typically 1: 1 to 2; preferably about 1:1.8 The reaction step a may be conducted at a temperature range of 0 to 80°C; preferably 10 to 70°C, and is allowed to proceed until substantially complete in from 2 to 20 hours; typically 8 to 16 hours.

We have found that in reaction step (d) at least a portion of the reactions react to produce, as an intermediate, the following epoxide A (wherein R is C₁₋₆alkyl): 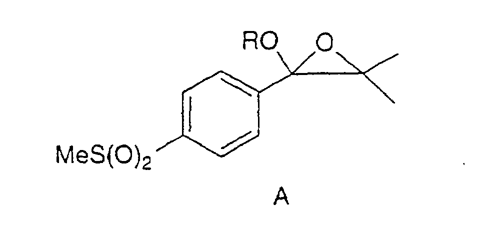 See JOC. 27, 4392 (1962); JACS. 76, 4402 (1954) and Chem. Ber. 116(11) 3631 (1983). Epoxide A is then converted to the compound of formula Ia by the reaction with a compound of formula 5a as described in step (d). Epoxide A may also be prepared by reacting a compound of formula 4 in a C₁₋₆alkanol solvent in the presence of a base such as K₂CO₃. Once again, see the references immediately above.

In an alternative aspect the compound of formula **3** and its chloro counterpart may be prepared by the following Friedel-Crafts reactions:

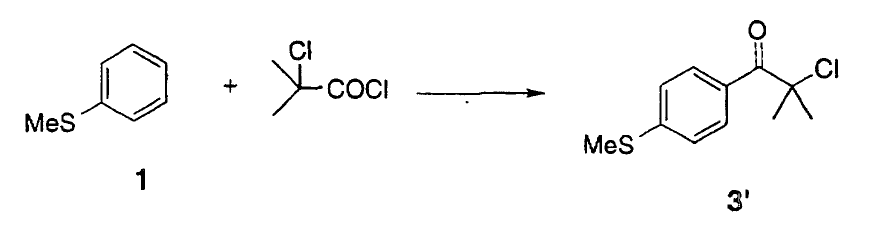

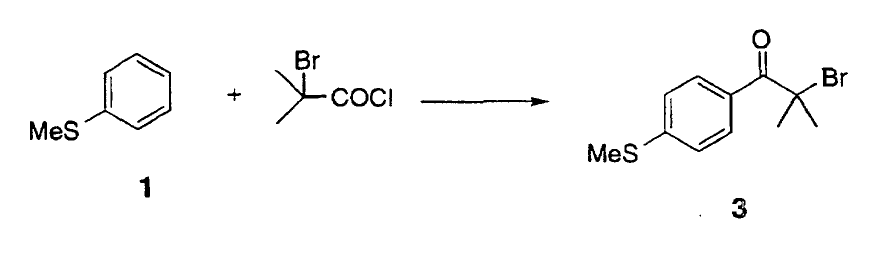

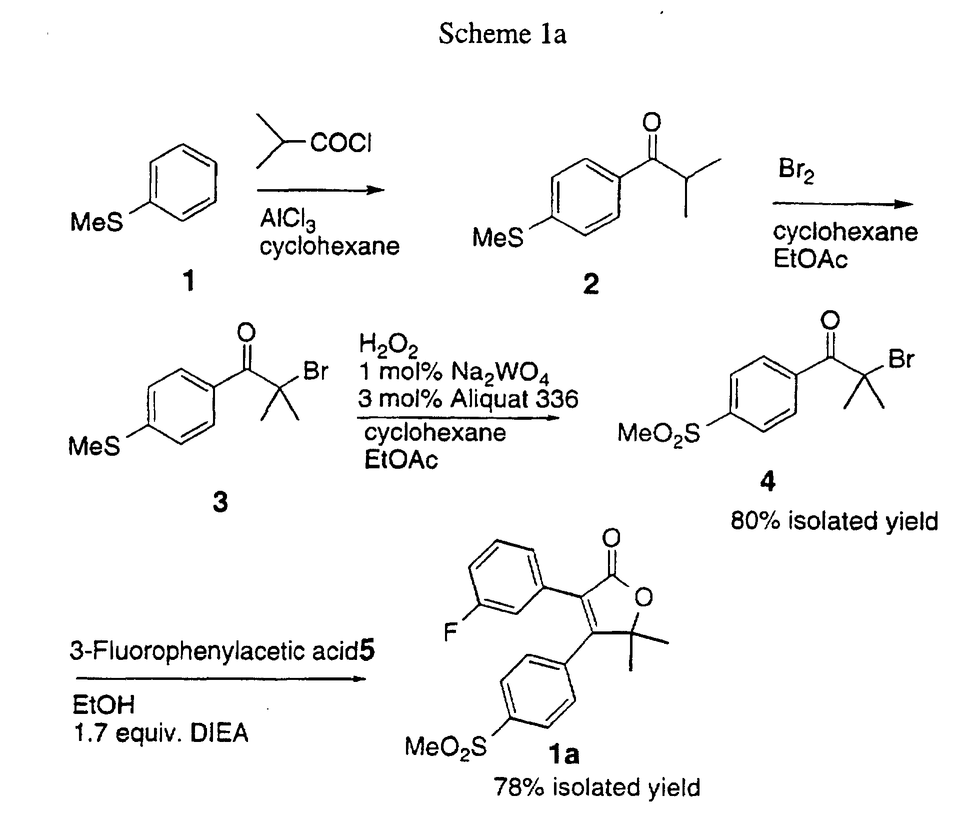

One alternative set of conditions for preparing compound **4** is depicted in Scheme 2a and Example 4.

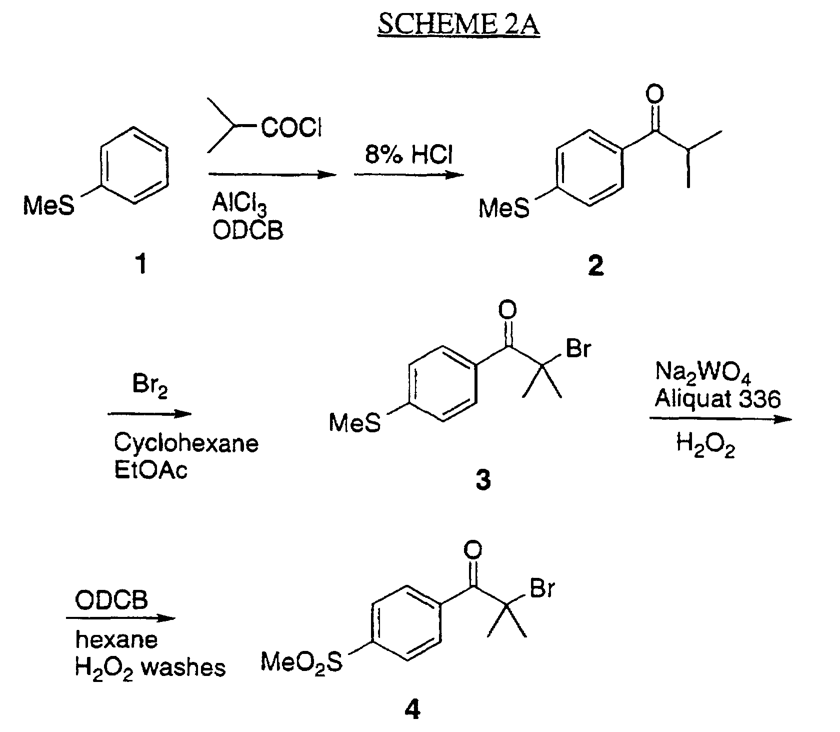

The Compound of Formula I and Ia is useful for the relief of pain, fever and inflammation of a variety of conditions including rheumatic fever, symptoms associated with influenza or other viral infections, common cold, low back and neck pain, dysmenorrhea, headache, toothache, sprains and strains, myositis, neuralgia, synovitis, arthritis, including rheumatoid arthritis degenerative joint diseases (osteoarthritis), gout and ankylosing spondylitis, bursitis, burns, injuries, following surgical and dental procedures. In addition, such a compound may inhibit cellular neoplastic transformations and metastic tumor growth and hence can be used in the treatment of cancer. Compounds of formula I may also be useful for the treatment of dementia including pre-senile and senile dementia, and in particular, dementia associated with Alzheimer Disease (ie Alzheimer's dementia).

By virtue of its high cyclooxygenase-2 (COX-2) activity and/or its selectivity for cyclooxygenase-2 over cyclooxygenase-1 (COX-1) as defined above, compounds of formula I and formula Ia will prove useful as an alternative to conventional non-steroidal antiinflammatory drugs (NSAID'S) particularly where such non-steroidal antiinflammatory drugs may be contra-indicated such as in patients with peptic ulcers, gastritis, regional enteritis, ulcerative colitis, diverticulitis or with a recurrent history of gastrointestinal lesions; GI bleeding, coagulation disorders including anemia such as hypoprothrombinemia, haemophilia or other bleeding problems (including those relating to reduced or impaired platelet function); kidney disease (eg impaired renal function); those prior to surgery or taking anticoagulants; and those susceptable to NSAID induced asthma.

Compounds of the present invention are inhibitors of cyclooxygenase-2 and are thereby useful in the treatment of cyclooxygenase-2 mediated diseases as enumerated above. This activity is illustrated by their ability to selectively inhibit cyclooxygenase-2 over cyclooxygenase-1. Accordingly, in one assay, the ability of the compounds of this invention to treat cyclooxygenase mediated diseases can be demonstrated by measuring the amount of prostaglandin E₂ (PGE₂) synthesized in the presence of arachidonic acid, cyclooxygenase-1 or cyclooxygenase-2 and a compound of formula I and formula Ia. The IC50 values represent the concentration of inhibitor required to return PGE₂ synthesis to 50 % of that obtained as compared to the uninhibited control. Illustrating this aspect, we have found that the Compounds of the Examples are more than 100 times more effective in inhibiting COX-2 than they are at inhibiting COX-1. In addition they all have a COX-2 IC50 of 1 nM to 1 µM. By way of comparison, Ibuprofen has an IC50 for COX-2 of 1 µM, and Indomethacin has an IC50 for COX-2 of approximately 100 nM.

For the treatment of any of these cyclooxygenase mediated diseases, compounds of formula I and formula Ia may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, cattle sheep, dogs, cats, etc., the compound of the invention is effective in the treatment of humans.

The invention will now be illustrated by the following nonlimiting examples in which, unless stated otherwise:
(i) all operations were carried out at room or ambient temperature, that is, at a temperature in the range 18-25°C; evaporation of solvent was carried out using a rotary evaporator under reduced pressure (600-4000 pascals: 4.5-30 mm. Hg) with a bath temperature of up to 60°C; the course of reactions was followed by thin layer chromatography (TLC) or High Pressure Liquid Chromatography (HPLC) and reaction times are given for illustration only; melting points are uncorrected and 'd' indicates decomposition; the melting points given are those obtained for the materials prepared as described; polymorphism may result in isolation of materials with different melting points in some preparations; the structure and purity of all final products were assured by at least one of the following techniques: TLC, mass spectrometry, nuclear magnetic resonance (NMR) spectrometry or microanalytical data; yields are given for illustration only; when given, NMR data is in the form of delta (δ) values for major diagnostic protons, given in parts per million (ppm) relative to tetramethylsilane (TMS) as internal standard, determined at 300 MHz or 400 MHz using the indicated solvent; conventional abbreviations used for signal shape are: s. singlet; d. doublet; t. triplet; m. multiplet; br. broad; etc.: in addition "Ar" signifies an aromatic signal; chemical symbols have their usual meanings; the following abbreviations have also been used v (volume), w (weight), b.p. (boiling point), m.p. (melting point), L (liter(s)), mL (milliliters), g (gram(s)), mg (milligrams(s)), mol (moles), mmol (millimoles), eq (equivalent(s)).

The following abbreviations have the indicated meanings:

| Alkyl Group Abbreviations | |
|---|---|
| Me = | methyl |
| Et = | ethyl |
| n-Pr = | normal propyl |
| i-Pr = | isopropyl |
| n-Bu = | normal butyl |
| i-Bu = | isobutyl |
| s-Bu = | secondary butyl |
| t-Bu = | tertiary butyl |
| c-Pr = | cyclopropyl |
| c-Bu = | cyclobutyl |
| c-Pen = | cyclopentyl |
| c-Hex = | cyclohexyl |

### EXAMPLE 1

| 4-Thiomethyl-isobutyrophenone **2** | |
|---|---|
| Thioanisole **1** (MW = 124.2 d = 1.058) | 312 g (2.51 mol) |
| Isobutyryl chloride (MW = 106.55, d = 1.017) | 286 mL (2.61 mol) |
| Aluminum chloride (98%, MW = 133.34) | 345 g (2.59 mol) |
| ortho-dichlorobenzene | 1 L |

A 5 L flask was charged under N2 with AlCl3 and ODCB. The vigorously stirred slurry was cooled to 8°C and isobutyryl chloride was added over 30 min., keeping the temperature at 10-15°C.

The addition of isobutyryl chloride was slightly exothermic.

The AlCl₃/isobutyryl chloride complex was aged at 7°C for 30 min. Efficient cooling was applied and thioanisole **1** was added to the reaction mixture over 120 min., maintaining an internal temperature of 8-13°C.

The addition of thioanisole was very exothermic. After the addition of about half of **1** a heavy yellow precipitate formed. The precipitation was accompanied by an exotherm. Gaseous HCl is formed in the reaction, so that the effluent gas stream should be scrubbed with aqueous NaOH before release into the atmosphere.

The reaction was warmed to 16°C over 1h.

The reaction mixture was a thick yellow slurry at this point. HPLC analysis of a quenched (EtOAc/H₂O) aliquot indicated completion of reaction.

The reaction mixture was cooled to 10°C and 1.6 L of 5% aqueous HCl were added over 45 min.

The addition was extremely exothermic and especially the initial addition required careful temperature monitoring.

The biphasic mixture was vigorously stirred for 60 min. The lower organic phase was removed.

A quantitative assay of the organic phase indicated a 98% yield.

| 4-thiomethyl-α-bromoisobutyrophenone **3** | |
|---|---|
| 4-Thiomethyl-isobutyrophenone **2** in ODCB | 2.46 mol |

Bromine (MW = 159.8, d - 3.102133 mL (2.58 mol)

A 5 L flask was charged with the solution of **2**. Approximately 10% of the bromine were added and the reaction mixture was stirred until the red color had dissipated after 45 min. The remainder of the Br₂ was added over 60 min.

The reaction was exothermic and the temperature rose to ca. 32°C.

Gaseous HBr was released from the reaction, thus the effluent gas stream was scrubbed with aqueous NaOH before release into the atmosphere.

The reaction mixture was aged for 2 h at 30°C when HPLC analysis indicated completion of the reaction

Addition of a slight excess of Brs leads to the partial oxidation of the sulfide to the sulfoxide .

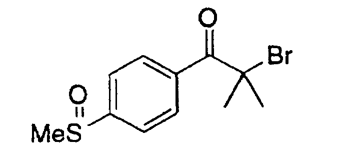

The reaction was quenched by the addition of 1.6 L H₂O and the resulting 1820 mL of organic phase were used directly for the oxidation (95% assay yield).

| 4-Methylsulfonyl-α-bromoisobutyrophenone **4** | |
|---|---|
| 4-Thiomethyl-α-bromoisobutyrophenone **3** in ODCB | 990 mL solution, (ca. 1.27 mol) |
| Sodium tungstate dihdrate (MW = 329.86) | 4.50 g (0.014 mol) |
| Aliquat 336 (MW = 404) | 22 g (0.054 mol) |
| Hydrogen peroxide, 30% (MW = 34) | 390 mL (3.44 mol) |

To a solution of **3** in ODCB in a 2 L reaction vessel with heating jacket, reflux condenser and bottom valve was added under N₂ as solution of Na₂WO₄ and Aliquat 336 in 30 mL H₂O. The heterogeneous reaction mixture was heated with vigorous stirring to 35°C and ca. 30 mL of H₂O₂ were added.

The oxidation was extremely exothermic. After an induction period of ca. 3 min. the temperature rose quickly to 50-65°C.

The remainder of the H₂O₂ was added over 1 h. At the end of the addition HPLC analysis indicated completion of the reaction.

The reaction mixture was heated to 80°C, the lower organic phase was removed and cooled to 6°C over 1 h.

The product precipitated at ca. 50°C without seeding.

The slurry was filtered and washed with 250 mL of ODCB and 300 mL of hexane and three times with 200 mL of 60°C H₂O. After drying 378 g of 4 (97% yield, ca. 91% overall yield from thioanisole) were obtained as a white powder.

| Preparation of isopropoxyacetic acid | | | |
|---|---|---|---|
| Material | amount | mol | equiv |
| Sodium chloroacetate (98%) | 159 g | 1.34 | 1.00 |
| Sodium hydroxide (97%) | 60 g | 1.45 | 1.08 |
| Isopropanol | 6 L | | |
| Conc. hydrochloric acid (∼12.1 M) | 125 mL | 1.50 | 1.12 |
| Sodium Chloride | ∼90 g | | |
| t-butylmethyl ether (MTBE) | 2.5 L | | |
| Toluene | 0.6 L | | |
| Product | | | |
| Isopropoxyacetic acid | 158 g | 1.34 | 1.00 |

A 5-L vessel fitted with a mechanical stirrer, thermocouple probe, and nitrogen inlet is charged with 2.0 L of isopropanol (K.F. 220 µg/mL) and sodium hydroxide (60 g, 1.45 mol). The mixture was heated at reflux until the solid sodium hydroxide dissolved.

A homogeneous solution is resulted after reflux for 3 h.

The solution was cooled at ∼ 70 °C and toluene (150 mL) was added. It was distilled until ∼ 1 L of distillate was collected. A mixture of IPA/toluene (85:15, 1 L) was added and ∼ 1L of liquid was distilled off (repeated 3 x). At the end of distillation (∼4 L of distillate collected), the solution was diluted with IPA to a volume of ~ 3.0 L.

The distillate is assayed to determine the amount of water removed. If the water removed is < 75% of the theoretical amount (K.F. of IPA + that in NaOH + 1 equiv generated), the distillation should be continued. The solution was then cooled at 60-70 °C and sodium chloroacetate (159 g, 1.34 mol) was added in portions over 5 min. No exotherm is observed during the addition.

The mixture was heated at reflux for 3 h and a sample of the slurry was taken for assay.

The reaction is followed by ¹HNMR. An aliquot (∼0.2 mL) of the mixture is taken and evaporated to dryness. The residue is dissolved in D₂O for ¹HNMR measurement. The reaction is considered completed whenthe starting material is < 3 % *vs*. product.

| | |
|---|---|
| δ 3.82 (s), i-PrO-CH₂-CO₂⁻ | product |
| δ 4.03 (s), Cl-CH₂-CO₂⁻ | starting material |
| δ 3.90 (s), HO-CH₂-CO₂⁻ | hydrolysis product |

The reaction was quenched by addition of 600 mL of water and concentrated under reduced pressure (150-200 mBar, 50-60 °C) until ∼2.5 L of distillated was collected. More water was added (400 mL) and the solution was distilled at normal pressure until the batch temperature reached ~103 °C (∼ 1 L of solution left, ∼3 L of solvent removed). The solution was cooled at 10-20 °C and neutralized by addition of conc. hydrochloric acid (125 mL, 1.5 mol).

External cooling may be needed during the addition of acid. The final pH should be > 2.3, preferably ∼2.
MTBE (800 mL) was added. The aqueous solution was saturated with sodium chloride (∼ 90 g) and the two-phase mixture was agitated for 0.5 h at 10-15 °C. The layers were separated and the aqueous layer was back extracted with 2 x 600 mL of MTBE. The organic layers were combined and washed with 2 x 100 mL of saturated aqueous sodium chloride.

The pH of the 2nd brine wash should be > 2.5.

The solution was dried over 4A molecular sieves (100 g) for 14 h and filtered. The sieves were washed with 3 x 150 mL of MTBE. MTBE was removed under reduced pressure (∼200 mBar, 45-50 °C). Isopropoxyacetic acid was obtained as a slightly yellow liquid.
Yield: 118 g, 75 % yield corrected for remaining MTBE (¹HNMR).

### Esterification

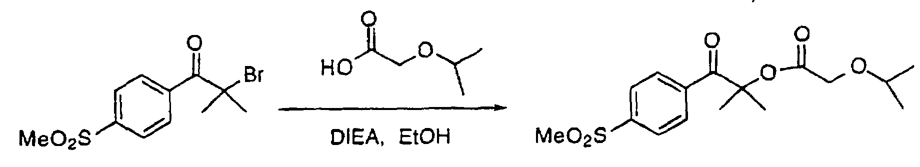

| Material | amount | mol | equiv |
|---|---|---|---|
| Bromosulfone | 50.0 g | .16 | 1.00 |
| Isopropoxyacetic acid | 23.2 g | .20 | 1.20 |
| Diisopropylethylamine | 48.5 mL | .28 | 1.70 |
| Ethanol (200 proof) | 450 mL | | |
| Product | | | |
| Ester | 56.0 g | .16 | 1.00 |

A 1L flask fitted with a mechanical stirrer, nitrogen inlet, and thermocouple was sequentially charged with dry ethanol (450 mL, K.F. < 100 µg/mL), isopropoxyacetic acid (23.2 g) , diisopropylethylamine (48.5 mL) and the bromosulfone (50.0 g). The mixture is heated to reflux until the bromosulfone is not detected (HPLC, reaction time 12-14 hours).

The reaction is considered complete when the bromosulfone is < 0.05 A% vs. product.

After the reaction was complete, the solution was allowed to cool and seeded at 42°C. Crystallization initiated immediately and the mixture was cooled to 1° C and aged 1 hr. The product ester is filtered and washed with ethanol (0°C 50 mL wash). After drying in a vacuum oven, the white crystalline material was used as is in the next step.
Yield: 41.5 g, 74%. Purity: 99 A% at 210 nm. ML losses are typically 4-6%.

### Cyclization of ester 6

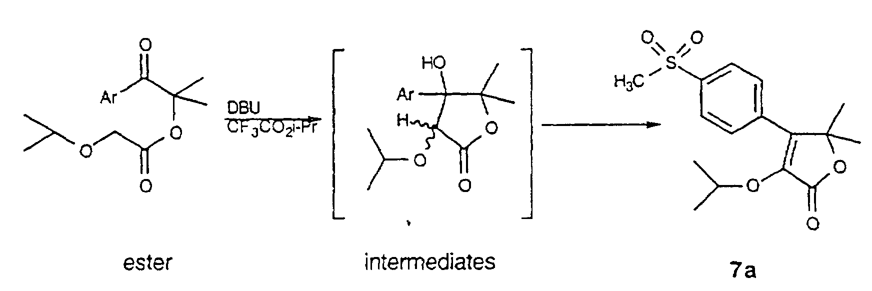

| Material | amount | mol | equiv |
|---|---|---|---|
| Ester | 5500 g | 16.1 | 1.00 |
| DBU | 3670 g | 24.1 | 1.50 |
| Isopropyl trifluoroacetate | 3010 g | 19.3 | 1.20 |
| Acetonitrile | 35 L | | |
| Water | 60 L | | |
| Product | | | |
| 7a | 5220 g | 16.1 | 1.00 |

A 100-L vessel fitted with a mechanical stirrer, nitrogen inlet, and thermocouple was sequentially charged with dry acetonitrile (32.2 L, K.F. < 100 µg/mL), isopropyl trifluoroacetate (3010 g, 19.3 mol), and DBU (3670 g, 24.1 mol). The solution was stirred at ∼20 °C for 15 min and the ester (5500 g, 16.1 mol) was added. The solution was heated at reflux under nitrogen and the progress of the reaction was followed by HPLC.

The reaction is considered complete when the intermediate peaks are < 0.2 A% vs. product.

After the reaction was complete, the solution was cooled at ∼40 °C and filtered (1 µ in-line capsule). The solution was then concentrated at 40-50 °C under reduced pressure until ∼20 L of distillate was collected. Water (35 L) was added slowly at ∼45 °C. After ∼13 L of water was added, the solution turned cloudy (40-45 °C) and ∼2 g of crystalline Ia was added as the seed. The mixture was aged for 30 min and the remaining water was added. The mixture was aged at ∼20 °C for 6 h then filtered. The cake was washed with 2 x 6.5 L of 1:4 MeCN/water and 3 x 6.5 L of water. The product was air dried and dried *in vacuo* (35 °C, 200 mBar).
Yield: ∼4800 g, 92%. Purity: > 99.9 LCAP at 210 nm. TG: 0.1%.

### EXAMPLE 2 (Alternative Preparation Of Compound 4)

| 4-Thiomethoxy-isobutyrophenone **2** | |
|---|---|
| Thioanisole **1** (MW = 124.2, d = 1.058) | 2.34 Kg (18.86 mol) |
| Isobutyryl chloride (MW = 106.55, d = 1.017) | 2.421 Kg (22.73 mol) |
| Aluminum chloride (98%, MW = 133.34) | 3.03 Kg (22.73 mol) |
| Cyclohexane | 20 L |

A 100 L reactor with internal cooling/heating coils and bottom release valve was charged under N₂ with AlCl₃ and cyclohexane. The vigorously stirred slurry was cooled to 7°C and isobutyryl chloride was added over 30 min., keeping the temperature at 7 - 12°C.

The addition of isobutyryl chloride was slightly exothermic and led to the formation of a clear solution.

The AlCl₃/isobutyryl chloride complex was aged at 10°C for 30 min. Efficient cooling was applied and thioanisole **1** was added to the reaction mixture over 30 min., maintaining the internal temperature of 8 - 12°C.

The addition of thioanisole was very exothermic and led to the formation of a deeply yellow slurry. The acylation was accompanied by the release of gaseous HCl, so that the effluent gas stream was scrubbed with aqueous NaOH before release into the atmosphere. The reaction did not proceed at an appreciable rate at temperatures below ca. 5°C.

The reaction was warmed to 19°C over 2 h and was then aged for an additional 2 h at 22°C.

The reaction mixture was a thick yellow slurry at this point HPLC analysis of a quenched (EtOAc/H₂O) aliquot indicated completion of reaction.

The reaction mixture was cooled to 10°C and 8 L of H₂O was added over 45 min.

The addition was extremely exothermic and especially the initial addition required careful temperature monitoring.

To the reaction vessel were added 32 L of EtOAc and the reaction mixture was stirred for 30 min. The aqueous phase was removed and the organic phase was washed twice with 8 L of ca. 1N aqueous HCl and once with 8 L of H₂O. The organic solution was dried over Na₂SO₄ for the next reaction.

| 4-Thiomethoxy-α-bromobutyrophenone **3** | |
|---|---|
| 4-Thiomethoxy-butyrophenone **2** in 32 L of cyclohexane and 32 L of EtOAc | ca. 31 mol |
| Bromine (MW = 159.8, d = 3.102) | 5.20 Kg (32.5 mol) |

A 100 L reactor with internal cooling/heating coils and bottom valve was charged with the solution of **2.** Bromine was added slowly over 90 min. to the solution.

The reaction was exothermic and the temperature rose to a. 35°C. The bromination was very slow at temperatures below 15°C.

Gaseous HBr was released from the reaction, thus the effluent gas stream was scrubbed with aqueous NaOH before release into the atmosphere.

It was not necessary to dry the EtOAc/cyclohexane solution of **2** prior to the bromination. It was found that the formation of **3** in a H₂O saturated system proceeded equally well, and that excess Br₂ oxidized the sulfide to the sulfoxide.

At the end of the addition, HPLC analysis indicated completion of the reaction.

The reaction mixture was washed with 15 L of H₂O, 8 L of saturated aqueous NaHCO₃ solution and twice with 4 L of H₂O.

| 4-Methylsulfonyl-α-bromoisobutyrophenone **4** | |
|---|---|
| 4-Thiomethoxy-α-bromobutyrophenone **3** in ca. 16 L of cyclohexane and 16 L of EtOAc | ca. 15.5 mol |
| Sodium tungstate dihydrate (MW = 329.86) | 51 g (0.155 mol) |
| Aliquat 336 (MW = 404) | 190 g (0.47 mol) |
| Hydrogen peroxide, 30% (MW = 34) | 4.8 L (42.5 mol) |

To a solution of **3** in EtOAc and cyclohexane in a 100 L reaction vessel with internal heating/cooling coils, double reflux condensers and bottom valve was added under N₂ 2 L of H₂O, Na₂WO₄ and Aliquat 336. The heterogeneous reaction mixture was heated with vigorous stirring to 59°C and ca. 300 mL of H₂O₂ were added.

The oxidation was extremely exothermic. After an induction period of ca. 3 min. the temperature rose quickly to 65°C, at which point reflux began.
The remainder of the H₂O₂ was added over 1 h at a rate that maintained a gentle reflux. At the end of the addition the reaction mixture was aged at 62°C for 1 h, when HPLC analysis indicated completion of the reaction.

The aqueous phase was removed at 62°C and the organic phase was washed twice at 62°C with 4 L H₂O. At atmospheric pressure, 5 L of solvent were removed by distillation from the solution.

The internal temperature rose to 75°C during the distillation. The distillation azeotropically dried the solution, and thus simplified the drying of the isolated **4**.
The solution was cooled to 10°C over 3 h.

The product precipitated at ca. 50°C without seeding.

The slurry was filtered and washed with 8 L of EtOAc/cyclohexane 50/50 and 8 L of cyclohexane. After drying in a N₂ stream, 3.79 Kg of **4** were obtained as a white powder.

Processing of an equal size batch using the same conditions gave 3.75 Kg of **4**.

### EXAMPLE 3

| 4-Thiomethoxy-isobutyrophenone **2** | |
|---|---|
| Thioanisole **1** (MW = 124.2, d = 1.058) | 2.34 Kg (18.86 mol) |
| Isobutyryl chloride (MW = 106.55, d = 1.017) | 2.421 Kg (22.73 mol) |
| Aluminum chloride (98%, MW = 133.34) | 3.03 Kg (22.73 mol) |
| Cyclohexane | 20 L |

A 100 L reactor with internal cooling/heating coils and bottom release valve was charged under N₂ with AlCl₃ and cyclohexane. The vigorously stirred slurry was cooled to 7°C and isobutyryl chloride was added over 30 min., keeping the temperature at 7 - 12°C.

The addition of isobutyryl chloride was slightly exothermic and led to the formation of a clear solution.

The AlCl₃/isobutyryl chloride complex was aged at 10°C for 30 min. Efficient cooling was applied and thioanisole **1** was added to the reaction mixture over 30 min., maintaining the internal temperature of 8 - 12°C.

The addition of thioanisole was very exothermic and led to the formation of a deeply yellow slurry. The acylation was accompanied by the release of gaseous HCl, so that the effluent gas stream was scrubbed with aqueous NaOH before release into the atmosphere. The reaction did not proceed at an appreciable rate at temperatures below ca. 5°C.

The reaction was warmed to 19°C over 2 h and was then aged for an additional 2 h at 22°C.

The reaction mixture was a thick yellow slurry at this point HPLC analysis of a quenched (EtOAC/H₂O) aliquot indicated completion of reaction.

The reaction mixture was cooled to 10°C and 8 L of H₂O was added over 45 min.

The addition was extremely exothermic and especially the initial addition required careful temperature monitoring.

To the reaction vessel were added 32 L of EtOAc and the reaction mixture was stirred for 30 min. The aqueous phase was removed and the organic phase was washed twice with 8 L of ca. 1N aqueous HCl and once with 8 L of H₂O. The organic solution was dried over Na₂SO₄ for the next reaction.

| 4-Thiomethoxy-α-bromobutyrophenone **3** | |
|---|---|
| 4-Thiomethoxy-butyrophenone **2** in 32 L of cyclohexane and 32 L of EtOAc | ca. 31 mol |
| Bromine (MW = 159.8, d = 3.102) | 5.20 Kg (32.5 mol) |

A 100 L reactor with internal cooling/heating coils and bottom valve was charged with the solution of **2**. Bromine was added slowly over 90 min. to the solution.

The reaction was exothermic and the temperature rose to a. 35°C. The bromination was very slow at temperatures below 15°C.

Gaseous HBr was released from the reaction, thus the effluent gas stream was scrubbed with aqueous NaOH before release into the atmosphere.

It was not necessary to dry the EtOAc/cyclohexane solution of **2** prior to the bromination. It was found that the formation of **3** in a H**2**O saturated system proceeded equally well, and that excess Br₂ oxidized the sulfide to the sulfoxide.

At the end of the addition, HPLC analysis indicated completion of the reaction.

The reaction mixture was washed with 15 L of H₂O, 8 L of saturated aqueous NaHCO₃ solution and twice with 4 L of H₂O.

| 4-Methylsulfonyl-α-bromoisobutyrophenone **4** | |
|---|---|
| 4-Thiomethoxy-α-bromobutyrophenone **3** in ca. 16 L of cyclohexane and 16 L of EtOAc | ca. 15.5 mol |
| Sodium tungstate dihydrate (MW = 329.86) | 51 g (0.155 mol) |
| Aliquat 336 (MW = 404) | 190 g (0.47 mol) |
| Hydrogen peroxide, 30% (MW = 34) | 4.8 L (42.5 mol) |

To a solution of ₃ in EtOAc and cyclohexane in a 100 L reaction vessel with internal heating/cooling coils, double reflux condensers and bottom valve was added under N₂ 2 L of H₂O, Na₂WO₄ and Aliquat 336. The heterogeneous reaction mixture was heated with vigorous stirring to 59°C and ca. 300 mL of H₂O₂ were added.

The oxidation was extremely exothermic. After an induction period of ca. 3 min. the temperature rose quickly to 65°C, at which point reflux began.
The remainder of the H₂O₂ was added over 1 h at a rate that maintained a gentle reflux. At the end of the addition the reaction mixture was aged at 62°C for 1 h, when HPLC analysis indicated completion of the reaction.

The aqueous phase was removed at 62°C and the organic phase was washed twice at 62°C with 4 L H₂O. At atmospheric pressure, 5 L of solvent were removed by distillation from the solution.

The internal temperature rose to 75°C during the distillation. The distillation azeotropically dried the solution, and thus simplified the drying of the isolated **4**.
The solution was cooled to 10°C over 3 h.

The product precipitated at ca. 50°C without seeding.

The slurry was filtered and washed with 8 L of EtOAc/cyclohexane 50/50 and 8 L of cyclohexane. After drying in a N₂ stream, 3.79 Kg of **4** were obtained as a white powder.

Processing of an equal size batch using the same conditions gave 3.75 Kg of **4.**

| Compound **7a** | |
|---|---|
| 4-Methylsulfonyl-α-bromoisobutyrophenone **4** (MW = 305.2) | |
| | 3.63 Kg (11.9 mol) |
| 3-Fluorophenylacetic acid (MW = 154.1) **5a** | 2.02 Kg (13.1 mol) |
| Diisopropylethylamine (MW = 129.2, d = 0.742) | 3.52 L (20.2 mol) |
| Ethanol, punctilious 200 proof | 24 L |

A 50 L flask under N₂ was charged with **4** and **5a**. The EtOH and the DIEA were added at 22°C and the resulting slurry was heated to 72°C for 16 h.

The reaction turned homogeneous at 60°C. During the course of the reaction Compound **7a** precipitated from the reaction mixture.
HPLC analysis of the supernatant indicated completion of the reaction.

The solution was heated at reflux for 1 h and 1.2 L of H₂O were added over 30 min. The slurry was then cooled to 15°C over 6 h, filtered and washed with 8 L of EtOH/H₂O 95/5, 8 L of EtOH/H₂O 50/50 and 8 L of punctilious EtOH. The resulting white crystalline product was dried in a N₂ stream to give 3.34 Kg of Compound **7a** (78% yield).

### EXAMPLE 4

| 4-Thiomethyl-isobutyrophenone **2** | |
|---|---|
| Thioanisole **1** (MW = 124.2 d = 1.058) | 312 g (2.51 mol) |
| Isobutyryl chloride (MW = 106.55, d = 1.017) | 286 mL (2.61 mol) |
| Aluminum chloride (98%, MW = 133.34) | 345 g (2.59 mol) |
| ortho-dichlorobenzene | 1 L |

A 5 L flask was charged under N2 with AlCl3 and ODCB. The vigorously stirred slurry was cooled to 8°C and isobutyryl chloride was added over 30 min., keeping the temperature at 10-15°C.

The addition of isobutyryl chloride was slightly exothermic.

The AlCl₃/isobutyryl chloride complex was aged at 7°C for 30 min. Efficient cooling was applied and thioanisole **1** was added to the reaction mixture over 120 min., maintaining an internal temperature of 8-13°C.

The addition of thioanisole was very exothermic. After the addition of about half of **1** a heavy yellow precipitate formed. The precipitation was accompanied by an exotherm. Gaseous HCl is formed in the reaction, so that the effluent gas stream should be scrubbed with aqueous NaOH before release into the atmosphere.

The reaction was warmed to 16°C over 1h.

The reaction mixture was a thick yellow slurry at this point. HPLC analysis of a quenched (EtOAc/H₂O) aliquot indicated completion of reaction.

The reaction mixture was cooled to 10°C and 1.6 L of 5% aqueous HCl were added over 45 min.

The addition was extremely exothermic and especially the initial addition required careful temperature monitoring.

The biphasic mixture was vigorously stirred for 60 min. The lower organic phase was removed.

A quantitative assay of the organic phase indicated a 98% yield.

| 4-thiomethyl-α-bromoisobutyrophenone 3 | |
|---|---|
| 4-Thiomethyl-isobutyrophenone **2** in ODCB | 2.46 mol |

Bromine (MW = 159.8, d - 3.102133 mL (2.58 mol)

A 5 L flask was charged with the solution of **2.**
Approximately 10% of the bromine were added and the reaction mixture was stirred until the red color had dissipated after 45 min. The remainder of the Br₂ was added over 60 min.

The reaction was exothermic and the temperature rose to ca. 32°C.

Gaseous HBr was released from the reaction, thus the effluent gas stream was scrubbed with aqueous NaOH before release into the atmosphere.

The reaction mixture was aged for 2 h at 30°C when HPLC analysis indicated completion of the reaction

Addition of a slight excess of Br₂ leads to the partial oxidation of the sulfide to the sulfoxide **8**.

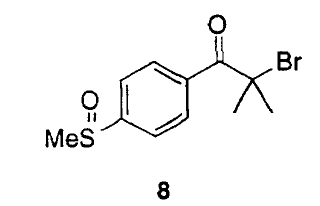

The reaction was quenched by the addition of 1.6 L H₂O and the resulting 1820 mL of organic phase were used directly for the oxidation (95% assay yield).

| 4-Methylsulfonyl-α-bromoisobutyrophenone **4** | |
|---|---|
| 4-Thiomethyl-α-bromoisobutyrophenone **3** | 990 mL solution, (ca. 1.27 in ODCB mol) |
| Sodium tungstate dihdrate (MW = 329.86) | 4.50 g (0.014 mol) |
| Aliquat 336 (MW = 404) | 22 g (0.054 mol) |
| Hydrogen peroxide, 30% (MW = 34) | 390 mL (3.44 mol) |

To a solution of **3** in ODCB in a 2 L reaction vessel with heating jacket, reflux condenser and bottom valve was added under N₂ as solution of Na₂WO₄ and Aliquat 336 in 30 mL H₂O. The heterogeneous reaction mixture was heated with vigorous stirring to 35°C and ca. 30 mL of H₂O₂ were added.

The oxidation was extremely exothermic. After an induction period of ca. 3 min. the temperature rose quickly to 50-65°C.

The remainder of the H₂O₂ was added over 1 h. At the end of the addition HPLC analysis indicated completion of the reaction.

The reaction mixture was heated to 80°C, the lower organic phase was removed and cooled to 6°C over 1 h.

The product precipitated at ca. 50°C without seeding.

The slurry was filtered and washed with 250 mL of ODCB and 300 mL of hexane and three times with 200 mL of 60°C H₂O.
After drying 378 g of 4 (97% yield, ca. 91% overall yield from thioanisole) were obtained as a white powder.

| **Compound 7a** | |
|---|---|
| 4-Methylsulfonyl-α-bromoisobutyrophenone **4** (MW = 305.2) | 28.37 g (0.093 mol) |
| 3-Fluorophenylacetic acid (MW = 154.1 ) **5a** | 15.8 g (0.102 mol) |
| Diisopropylethylamine (MW = 129.2, d = 0.742) | 28 mL (0.16 mol) |
| Ethanol, 2BAT | 160 mL |

A 500 mL flask under N₂ was charged with **4** and **5a**. The EtOH and the DIEA were added at 22°C and the resulting slurry was heated at 76°C for 14 h.

The reaction turned homogeneous at 60°C and the product precipitated during the age.

The slurry was cooled to 25°C over 4 h, filtered and washed with 150 mL of 2BAT EtOH. The resulting white crystalline product was dried in a N₂ stream to give 25.02 g of Compound **7a** (75% yield).

### EXAMPLE 5

### Compound A (R = ethyl)

To a solution of 4-methylsulfonyl-α-bromo isobutyrophenone (814mg, 2.67mmol) in 13 ml of ethanol is added 2.54g of solid K₂CO₃. The reaction mixture is filtered after 3 hours and the organic filtrate is evaporated to give 744mg of compound A (R = ethyl) as a clear oil.
¹H NMR (CDCl₃) 7.9 (2H, d), 7.5 (2H, d), 3.9 (1H, dt), 3.2 (1H,), 2.5 (3H, s), 1.5 (3H, s), 1.0 (3H, t), 0.8 (3H, s).

## Claims

1. A process for making compounds of Formula I 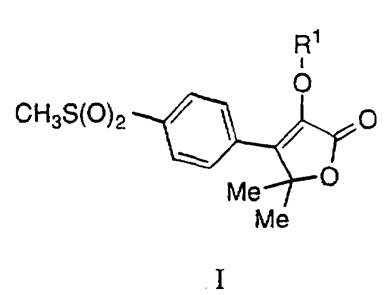
R¹ is selected from the group consisting of
(a) linear or branched C₁₋₆alkyl,
(b) mono-, di- or tri-substituted phenyl or naphthyl wherein the substituents are selected from the group consisting of
(1) hydrogen,
(2) halo,
(3) C₁₋₃alkoxy,
(4) CN,
(5) C₁₋₃ fluoroalkyl
(6) C₁₋₃ alkyl,
(7) -CO₂H,
the process comprising:
(a) reacting thioanisole 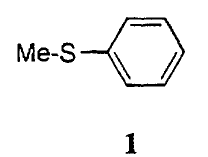 in a non-reactive solvent and in the presence of a Lewis Acid, with isobutyryl chloride 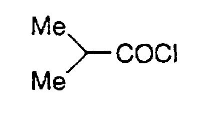 to yield compound **2** 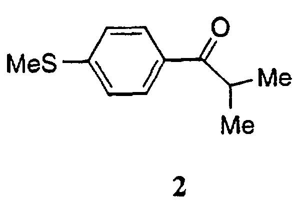
(b) brominating reacting a compound **2** with a brominating agent in a non-reactive solvent (as defined above), in the presence of a second solvent, such as ethyl acetate to yield compound **3** 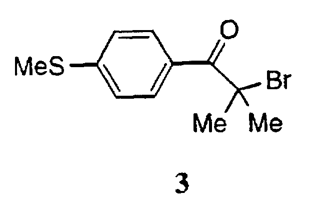
(c) oxidizing compound **3** in a non-reactive solvent (as defined above), with an oxidizing agent, optionally in the presence of a suitable catalyst to yield compound **4** 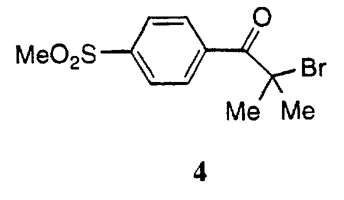
(d) reacting compound **4** in an alkanol solvent with compound **5** 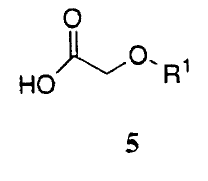 in the presence of a suitable base to yield a compound of formula **6** 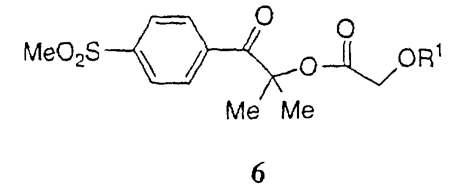
(e) reacting compound **6** in an aprotic solvent with a strong base to yield a cyclized compound of formula **7.** 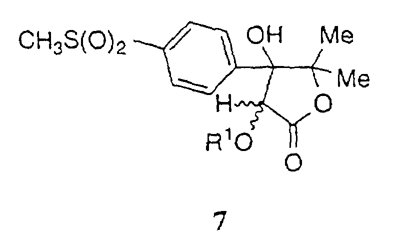 which after dehydration in the presence of a water scavenger yields a compound of formula I.

2. A process for making compounds of Formula I 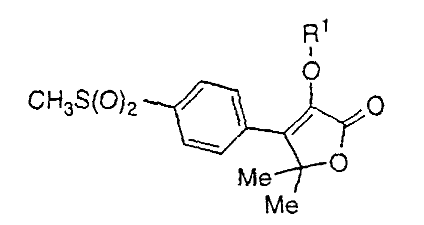
R¹ is selected from the group consisting of
(a) linear or branched C₁₋₆alkyl,
(b) mono-, di- or tri-substituted phenyl or naphthyl wherein the substituents are selected from the group consisting of
(1) hydrogen,
(2) halo,
(3) C₁₋₃alkoxy,
(4) CN,
(5) C₁₋₃ fluoroalkyl
(6) C₁₋₃ alkyl,
(7) -CO₂H,
comprising the steps of
(e) reacting compound **6** 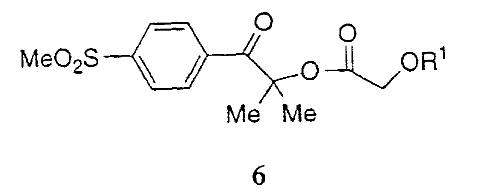 in an aprotic solvent with a strong base to yield a cyclized compound of formula **7.** 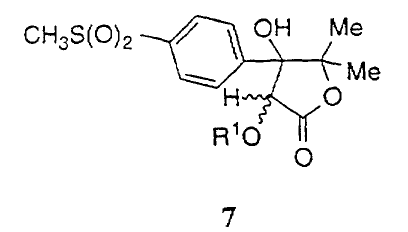 which after dehydration in the presence of a water scavenger yields a compound of formula I.

3. A process according to Claim 2 wherein the water scavenger is isopropyl trifluoroacetate.

4. A process according to Claim 2 wherein the aprotic solvent is acetonitrile.

5. A process according to Claim 2 wherein the strong base is 1,8-Diazabicyclo[5.4.0]undec-7-ene.

6. A process according to Claim 2 wherein the water scavenger is isopropyl trifluoroacetate; the aprotic solvent is acetonitrile; and the strong base is 1,8-Diazabicyclo[5.4.0]undec-7-ene.

7. A process according to Claim 6 wherein R¹ is isopropyl.

8. A process according to Claim 2 or 7 for making compounds of Formula I comprising the steps of
(d) reacting compound **4** 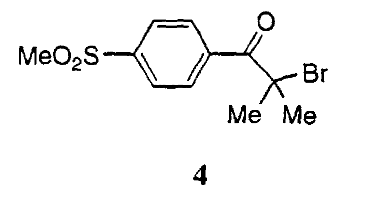 in an alkanol solvent with compound **5** 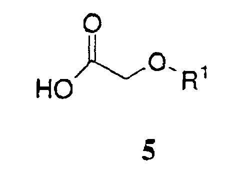 in the presence of a suitable base to yield a compound of formula **6** 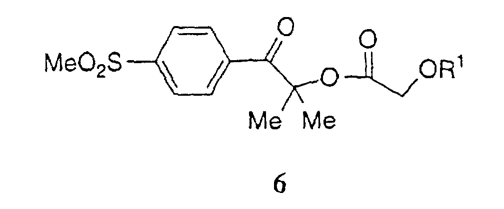
(e) reacting compound **6** in an aprotic solvent with a strong base to yield a cyclized compound of formula **7.** 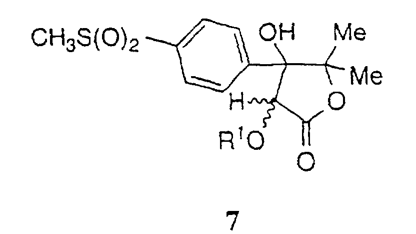 which after dehydration in the presence of a water scavenger yields a compound of formula I.

9. A process according to Claim 8 for making compounds of Formula I comprising the steps of I
(c) oxidizing compound **3** 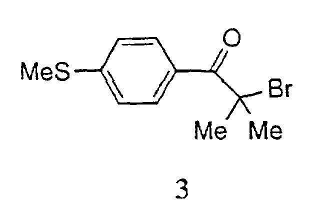 in a non-reactive solvent (as defined above), with an oxidizing agent, optionally in the presence of a suitable catalyst to yield compound **4** 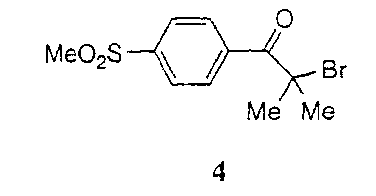
(d) reacting compound **4** in an alkanol solvent with compound **5** 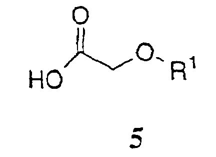 in the presence of a suitable base to yield a compound of formula **6** 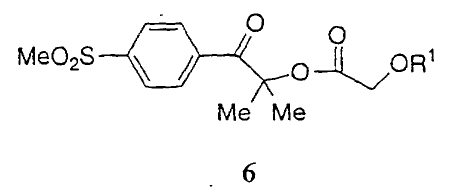
(e) reacting compound **6** in an aprotic solvent with a strong base to yield a cyclized compound of formula **7.** 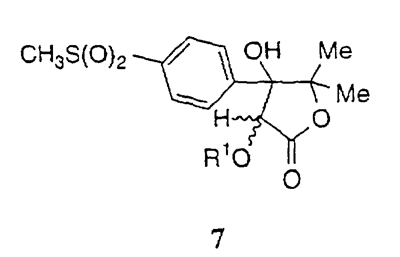 which after dehydration in the presence of a water scavenger yields a compound of formula I.

10. A process according to Claim 9 for making a compound of formula I comprising the steps of
(b) brominating reacting a compound **2** 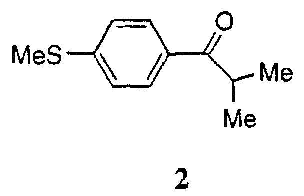 with a brominating agent in a non-reactive solvent, in the presence of a second solvent, such as ethyl acetate to yield compound **3** 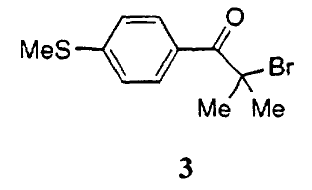
(c) oxidizing compound **3** in a non-reactive solvent (as defined above), with an oxidizing agent, optionally in the presence of a suitable catalyst to yield compound **4** 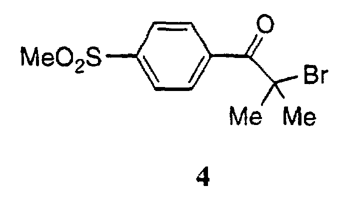
(d) reacting compound **4** in an alkanol solvent with compound **5** 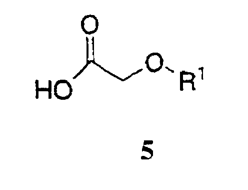 in the presence of a suitable base to yield a compound of formula **6** 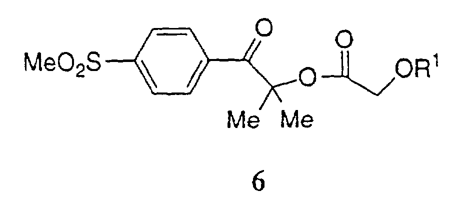
(e) reacting compound **6** in an aprotic solvent with a strong base to yield a cyclized compound of formula **7:** 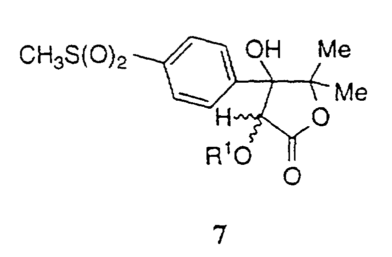 which after dehydration in the presence of a water scavenger yields a compound of formula I.

11. A compound of the Formulae 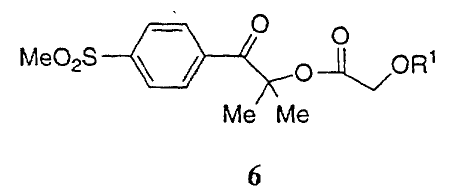 or 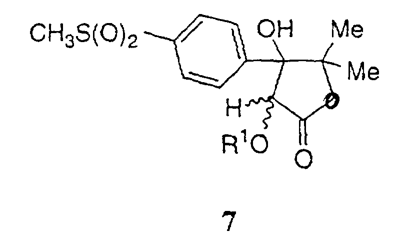 wherein
R¹ is selected from the group consisting of
(a) linear or branched C₁₋₆alkyl,
(b) mono-, di- or tri-substituted phenyl or naphthyl wherein the substituents are selected from the group consisting of
(1) hydrogen,
(2) halo,
(3) C₁₋₃alkoxy,
(4) CN,
(5) C₁₋₃ fluoroalkyl
(6) C₁₋₃ alkyl,
(7) -CO₂H,

12. A compound according to Claim 11 wherein R1 is isopropyl.

13. A process for making compounds of Formula Ia 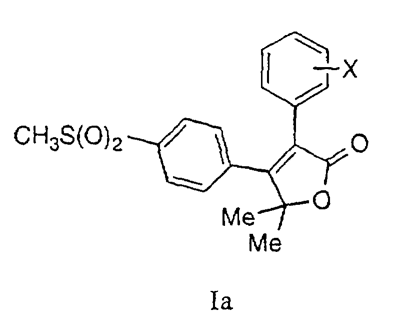 X is H, fluoro or chloro, wherein there are 1, 2 or 3 groups X; the process comprising:
(a) reacting thioanisole 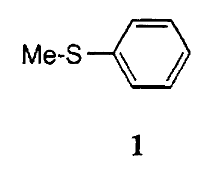 in a non-reactive solvent and in the presence of a Lewis Acid, with isobutyryl chloride 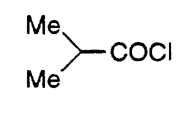 to yield compound **2** 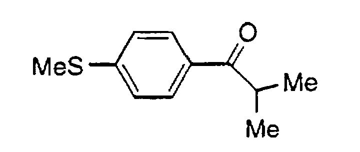
(b) brominating a compound **2** with a brominating agent in a non-reactive solvent (as defined above), in the presence of a second solvent to yield compound **3** 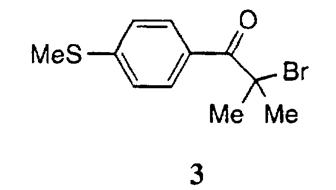 or chlorinating a compound **2** with chlorinating agent in a non-reactive solvent (as defined above), in the presence of a second solvent to yield compound **3a** 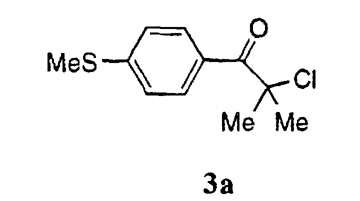
(c) oxidizing compound **3** or **3a** in a non-reactive solvent (as defined above), with an oxidizing agent, optionally in the presence of a suitable catalyst to yield compound **4** 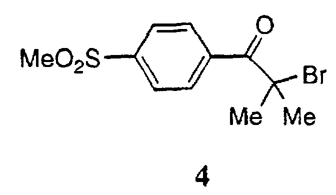 or 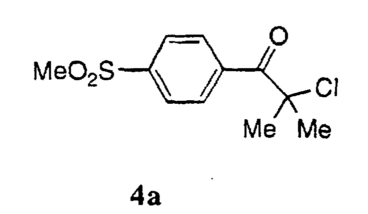
(d) reacting compound **4** or **4a** in an alkanol solvent with compound **5a** 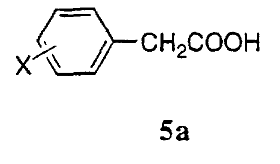 in the presence of a suitable base to yield a compound of Formula Ia

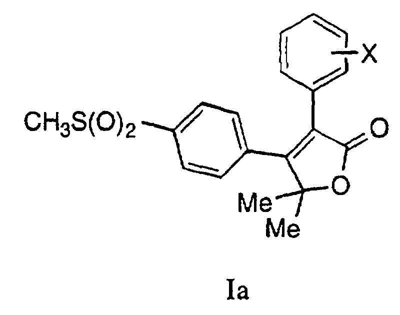

14. A process for making compounds of Formula Ia 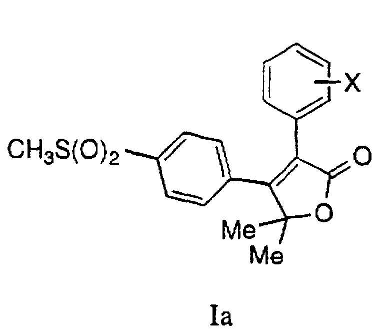
X is fluoro or chloro;
the process comprising:
(a) reacting thioanisole 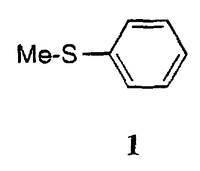 in a non-reactive solvent and in the presence of a Lewis Acid, with isobutyryl chloride 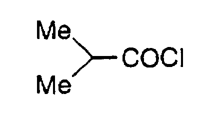 to yield compound **2**: 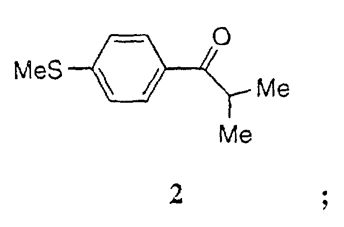
(b) reacting a compound **2:**
with a brominating agent in a non-reactive solvent, in the presence of a second solvent to yield compound **3:** 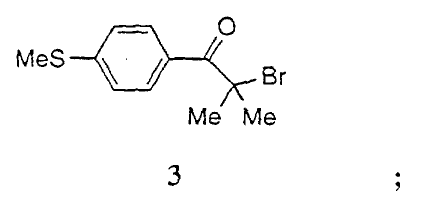
(c) oxidizing compound **3** in a non-reactive solvent, with an oxidizing agent, optionally in the presence of a suitable catalyst, to yield compound **4:** 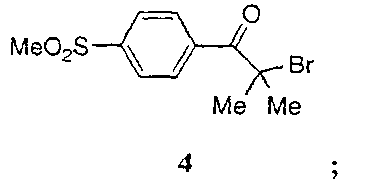
(d) reacting compound **4** in an alkanol solvent with compound **5a** 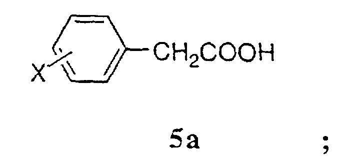
wherein X is F or Cl; in the presence of a suitable base to yield a compound of Formula Ia:

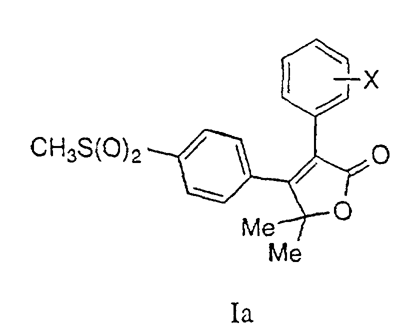

15. A process for making compounds of Formula Ia: 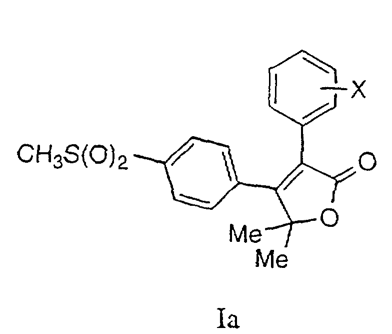
wherein X is fluoro or chloro;
comprising:
(d) reacting compound **4:** 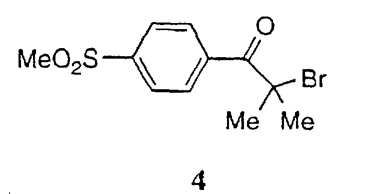 in an alkanol solvent with compound **5a** 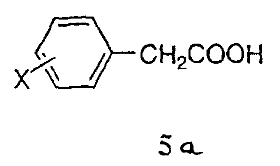 wherein X is F or Cl;
in the presence of a suitable base to yield a compound of Formula Ia

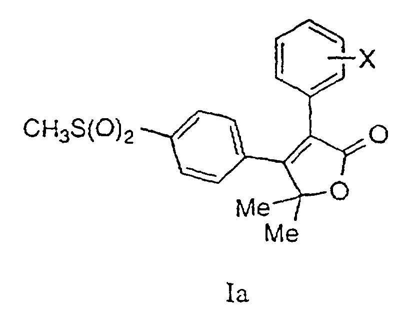

16. A process according to Claim 15 for making compounds of Formula Ia 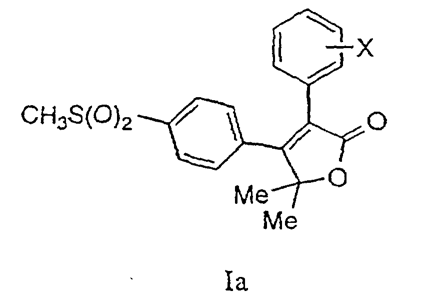 wherein X is fluoro or chloro,
comprising:
(c) oxidizing compound **3** 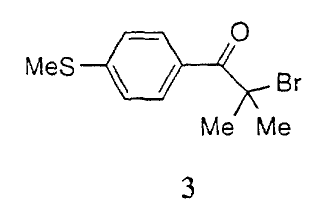 in a non-reactive solvent, with an oxidizing agent, optionally in the presence of a suitable catalyst to yield compound **4** 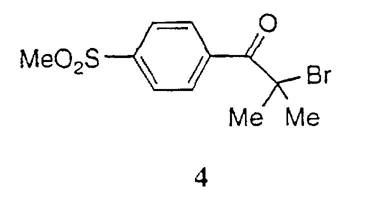
and (d) reacting compound **4** in an alkanol solvent with compound **5a** 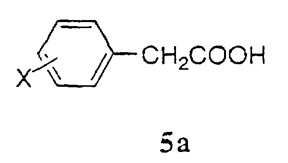 in the presence of a suitable base to yield a compound of formula Ia

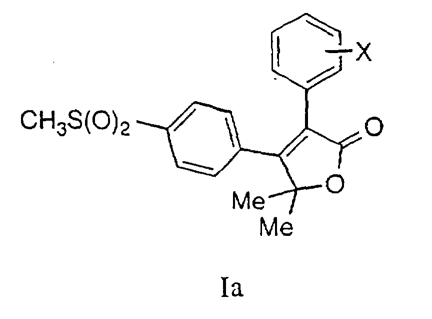

17. A process according to Claim 16 for making compounds of Formula Ia: 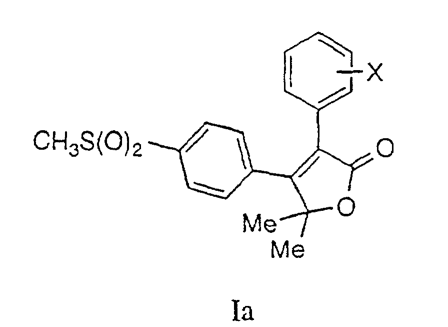 wherein X is fluoro or chloro,
comprising:
(b) reacting compound **2**: 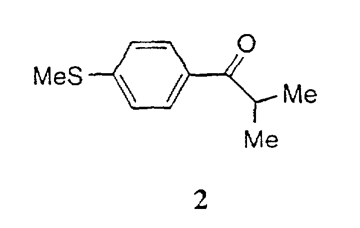 with a brominating agent in a non-reactive solvent, in the presence of a second solvent to yield compound **3:** 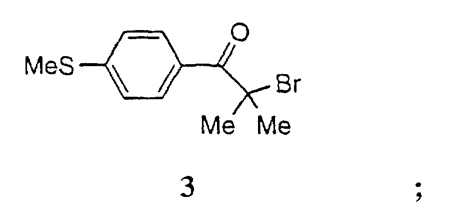
(c) oxidizing compound **3** in a non-reactive solvent with an oxidizing agent, optionally in the presence of a suitable catalyst, to yield compound **4:** 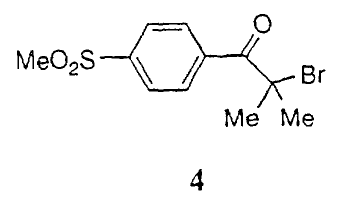
and (d) reacting compound **4** in an alkanol solvent with compound **5a** 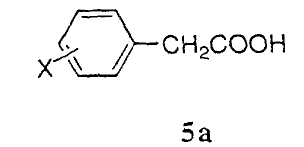 in the presence of a suitable base to yield a compound of formula Ia:

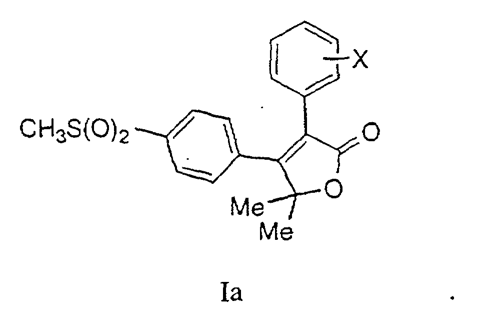

18. A process according to Claim 14, 15, 16 or 17 wherein X is 3-fluoro.

19. A process for making compounds of formula **4** or **4a:** 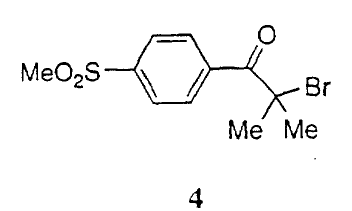 or 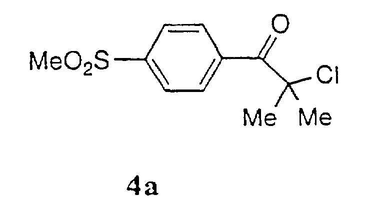 comprising:
(a) reacting thioanisole 1: 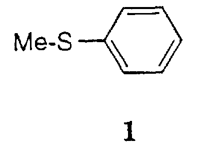 in a non-reactive solvent and in the presence of a Lewis Acid, with isobutyryl chloride: 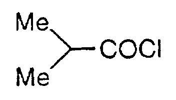 to yield compound **2** 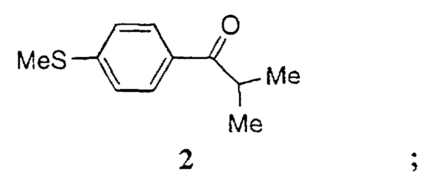
(b) reacting compound **2** with a brominating agent or with a chlorinating agent in a non-reactive solvent, in the presence of a second solvent, to yield a compound of formula **3** or **3a:** 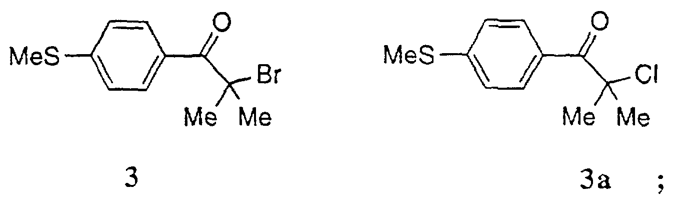
and (c) oxidizing compound **3** or **3a** in a non-reactive solvent (as defined above), with an oxidizing agent, optionally in the presence of a suitable catalyst to yield compound **4 or 4a:**

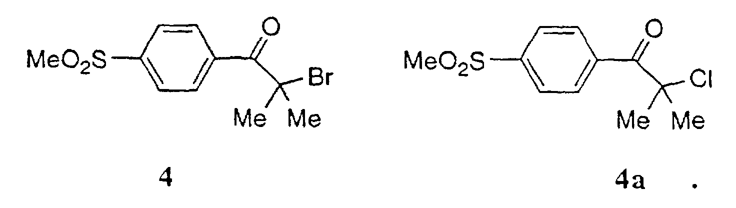

20. A process according to Claim 19 wherein the non-reactive solvent is cyclohexane or ortho-dichloro benzene.

21. A process according to Claim 19 wherein the Lewis acid is aluminum chloride.

22. A process according to Claim 19 wherein the brominating agent is bromine.

23. A process according to Claim 19 wherein the oxidizing agent is hydrogen peroxide.

24. A process according to Claim 19 wherein X is 3-fluoro.

25. A compound which is 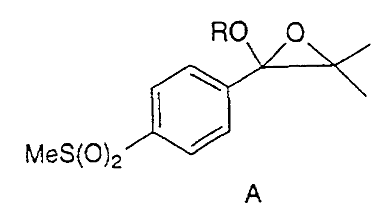 wherein R is C₁₋₆alkyl.

26. A compound according to Claim 25 wherein R is ethyl.

## Patentansprüche

1. Ein Verfahren zur Herstellung von Verbindungen der Formel I 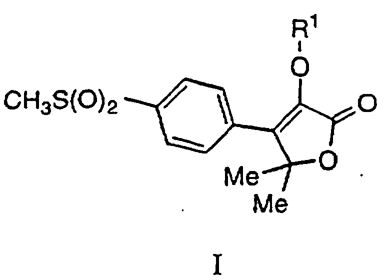
wobei R¹ ausgewählt ist aus der Gruppe, bestehend aus
(a) linearem oder verzweigtem C₁₋₆-Alkyl,
(b) mono-, di- oder trisubstituiertem Phenyl oder Naphthyl, wobei die Substituenten ausgewählt sind aus der Gruppe, bestehend aus
(1) Wasserstoff,
(2) Halogen,
(3) C₁₋₃-Alkoxy,
(4) CN,
(5) C₁₋₃-Fluoralkyl,
(6) C₁₋₃-Alkyl,
(7) -CO₂H,
wobei das Verfahren umfaßt:
(a) Umsetzung von Thioanisol 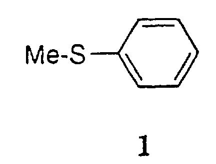 in einem nichtreaktiven Lösungsmittel und in Gegenwart einer Lewissäure mit Isobutyrylchlorid 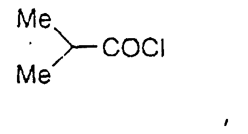 um Verbindung **2** zu ergeben 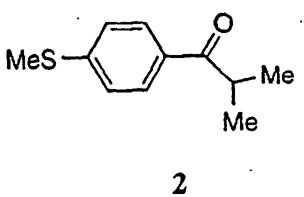
(b) Bromierungsumsetzung einer Verbindung **2** mit einem Bromierungsmittel in einem nichtreaktiven Lösungsmittel (wie es oben definiert ist) in Gegenwart eines zweiten Lösungsmittels, wie z.B. Ethylacetat, um Verbindung **3** zu ergeben 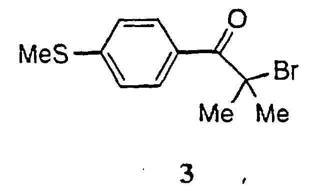
(c) Oxidation von Verbindung **3** in einem nichtreaktiven Lösungsmittel (wie es oben definiert ist) mit einem Oxidationsmittel, gegebenenfalls in Gegenwart eines geeigneten Katalysators, um Verbindung **4** zu ergeben 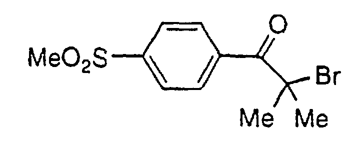
(d) Umsetzung von Verbindung **4** in einem Alkanol-Lösungsmittel mit Verbindung **5** 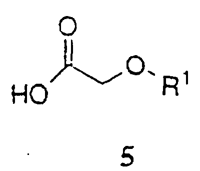 in Gegenwart einer geeigneten. Base, um eine Verbindung der Formel **6** zu ergeben 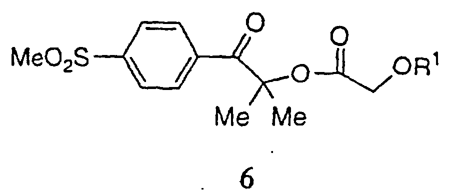
(e) Umsetzung von Verbindung **6** in einem aprotischen Lösungsmittel mit einer starken Base, um eine cyclisierte Verbindung der Formel **7** zu ergeben. 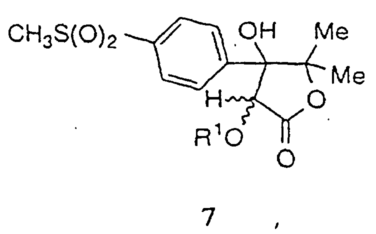 die nach der Dehydratisierung in Gegenwart eines Wasserfängers eine Verbindung der Formel I ergibt.

2. Ein Verfahren zur Herstellung von Verbindungen der Formel I 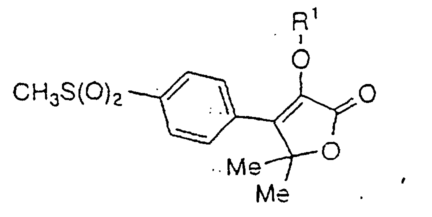
wobei R¹ ausgewählt ist aus der Gruppe, bestehend aus
(a) linearem oder verzweigtem C₁₋₆-Alkyl,
(b) mono-, di- oder trisubstituiertem Phenyl oder Naphthyl, wobei die Substituenten ausgewählt sind aus der Gruppe, bestehend aus
(1) Wasserstoff,
(2) Halogen,
(3) C₁₋₃-Alkoxy,
(4) CN,
(5) C₁₋₃-Fluoralkyl,
(6) C₁₋₃-Alkyl,
(7) -CO₂H,
umfassend die Schritte
(e) Umsetzung von Verbindung **6** 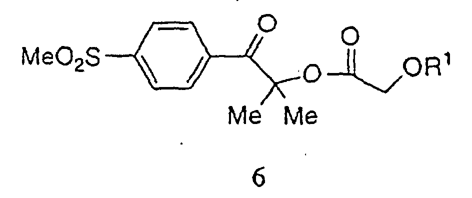 in einem aprotischen Lösungsmittel mit einer starken Base, um eine cyclisierte Verbindung der Formel **7** zu ergeben 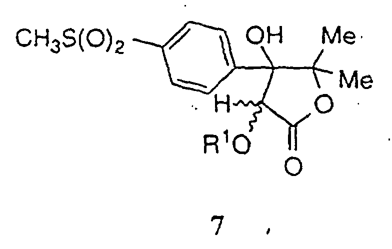 die nach der Dehydratisierung in Gegenwart eines Wasserfängers eine Verbindung der Formel I ergibt.

3. Ein Verfahren gemäß Anspruch 2, wobei der Wasserfänger Isopropyltrifluoracetat ist.

4. Ein Verfahren gemäß Anspruch 2, wobei das aprotische Lösungsmittel Acetonitril ist.

5. Ein Verfahren gemäß Anspruch 2, wobei die starke Base 1,8-Diazabicyclo[5.4.0]undec-7-en ist.

6. Ein Verfahren gemäß Anspruch 2, wobei der Wasserfänger Isopropyltrifluoracetat ist, das aprotische Lösungsmittel Acetonitril ist und die starke Base 1,8-Diazabicyclo[5.4.0]undec-7-en ist.

7. Ein Verfahren gemäß Anspruch 6, wobei R¹ Isopropyl ist.

8. Ein Verfahren gemäß Anspruch 2 oder 7 zur Herstellung von Verbindungen der Formel I, umfassend die Schritte
(d) Umsetzung von Verbindung **4** 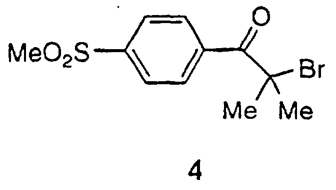 in einem Alkanol-Lösungsmittel mit Verbindung **5** 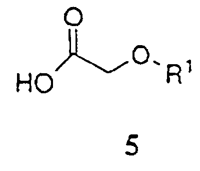 in Gegenwart einer geeigneten Base, um eine Verbindung der Formel **6** zu ergeben 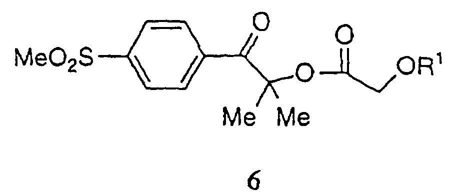
(e) Umsetzung von Verbindung **6** in einem aprotischen Lösungsmittel mit einer starken Base, um eine cyclisierte Verbindung der Formel **7** zu ergeben 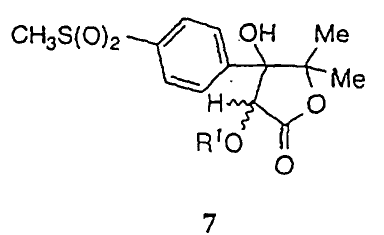 die nach der Dehydratisierung in Gegenwart eines Wasserfängers eine Verbindung der Formel I ergibt.

9. Ein Verfahren gemäß Anspruch 8 zur Herstellung von Verbindungen der Formel I, umfassend die Schritte
(c) Oxidation von Verbindung **3** 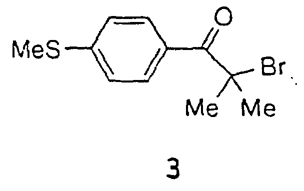 in einem nichtreaktiven Lösungsmittel (wie es oben definiert ist) mit einem Oxidationsmittel, gegebenenfalls in Gegenwart eines geeigneten Katalysators, um Verbindung **4** zu ergeben 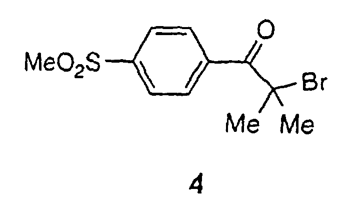
(d) Umsetzung von Verbindung **4** in einem Alkanol-Lösungsmittel mit Verbindung **5** 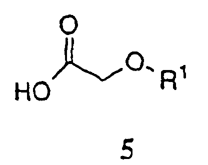 in Gegenwart einer geeigneten Base, um eine Verbindung der Formel **6** zu ergeben 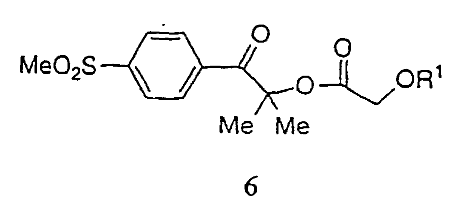
(e) Umsetzung von Verbindung **6** in einem aprotischen Lösungsmittel mit einer starken Base, um eine cyclisierte Verbindung der Formel **7** zu ergeben 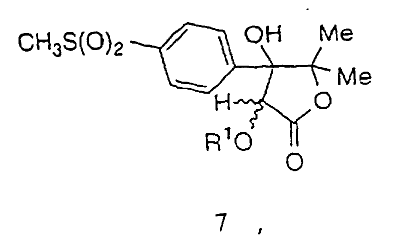 die nach der Dehydratisierung in Gegenwart eines Wasserfängers eine Verbindung der Formel I ergibt.

10. Ein Verfahren gemäß Anspruch 9 zur Herstellung einer Verbindung der Formel I, umfassend die Schritte
(b) Bromierungsumsetzung einer Verbindung **2** 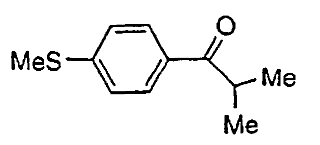 mit einem Bromierungsmittel in einem nichtreaktiven Lösungsmittel in Gegenwart eines zweiten Lösungsmittels, wie z.B. Ethylacetat, um Verbindung **3** zu ergeben 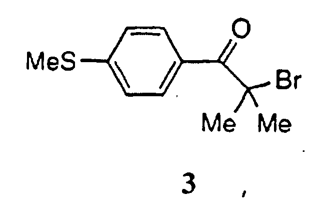
(c) Oxidation von Verbindung **3** in einem nichtreaktiven Lösungsmittel (wie es oben definiert ist) mit einem Oxidationsmittel, gegebenenfalls in Gegenwart eines geeigneten Katalysators, um Verbindung **4** zu ergeben 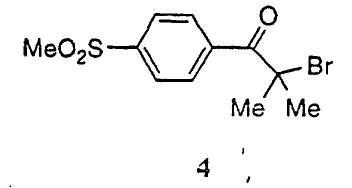
(d) Umsetzung von Verbindung **4** in einem Alkanol-Lösungsmittel mit Verbindung **5** 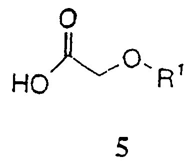 in Gegenwart einer geeigneten Base, um eine Verbindung der Formel **6** zu ergeben 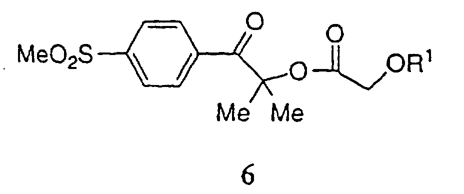
(e) Umsetzung von Verbindung **6** in einem aprotischen Lösungsmittel mit einer starken Base, um eine cyclisierte Verbindung der Formel **7** zu ergeben: 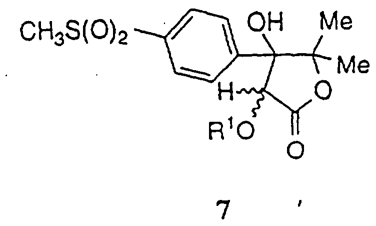 die nach der Dehydratisierung in Gegenwart eines Wasserfängers eine Verbindung der Formel I ergibt.

11. Eine Verbindung der Formeln 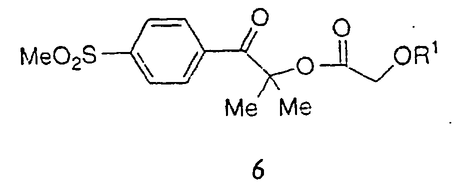 oder 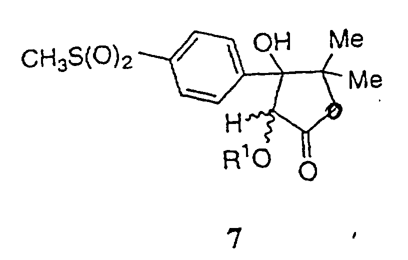 wobei
R¹ ausgewählt ist aus der Gruppe, bestehend aus
(a) linearem oder verzweigtem C₁₋₆-Alkyl,
(b) mono-, di- oder trisubstituiertem Phenyl oder Naphthyl, wobei die Substituenten ausgewählt sind aus der Gruppe, bestehend aus
(1) Wasserstoff,
(2) Halogen,
(3) C₁₋₃-Alkoxy,
(4) CN,
(5) C₁₋₃-Fluoralkyl,
(6) C₁₋₃-Alkyl,
(7) -CO₂H.

12. Eine Verbindung gemäß Anspruch 11, wobei R¹ Isopropyl ist.

13. Ein Verfahren zur Herstellung von Verbindungen der Formel Ia 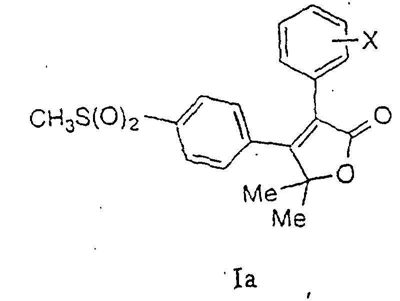 wobei X H, Fluor oder Chlor ist und es 1, 2 oder 3 Gruppen X gibt,
wobei das Verfahren umfaßt:
(a) Umsetzung von Thioanisol 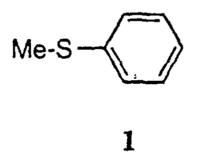 in einem nichtreaktiven Lösungsmittel und in Gegenwart einer Lewissäure mit Isobutyrylchlorid 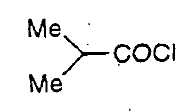 um Verbindung **2** zu ergeben 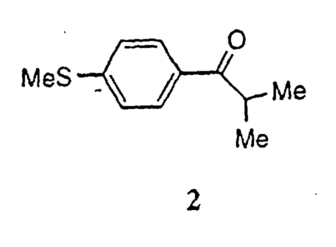
(b) Bromierung einer Verbindung **2** mit einem Bromierungsmittel in einem nichtreaktiven Lösungsmittel (wie es oben definiert ist) in Gegenwart eines zweiten Lösungsmittels, um Verbindung **3** zu ergeben 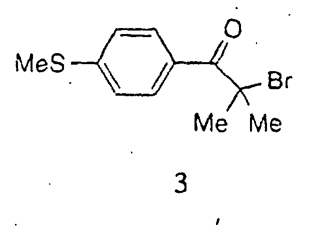 oder Chlorierung einer Verbindung **2** mit einem Chlorierungsmittel in einem nichtreaktiven Lösungsmittel (wie es oben definiert ist) in Gegenwart eines zweiten Lösungsmittels, um Verbindung **3a** zu ergeben 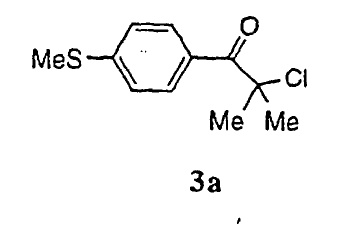
(c) Oxidation von Verbindung **3** oder **3a** in einem nichtreaktiven Lösungsmittel (wie es oben definiert ist) mit einem Oxidationsmittel, gegebenenfalls in Gegenwart eines geeigneten Katalysators, um Verbindung **4** zu ergeben 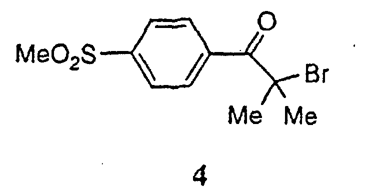 oder 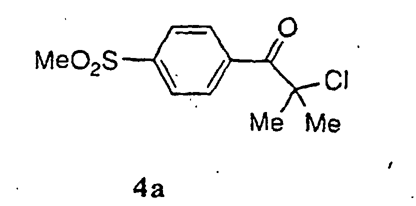
(d) Umsetzung von Verbindung **4** oder **4a** in einem Alkanol-Lösungsmittel mit Verbindung **5a** 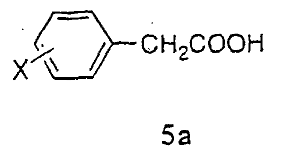 in Gegenwart einer geeigneten Base, um eine Verbindung der Formel Ia zu ergeben

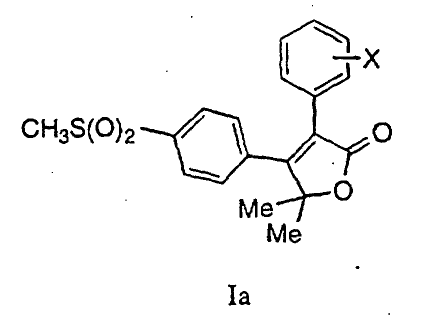

14. Ein Verfahren zur Herstellung von Verbindungen der Formel 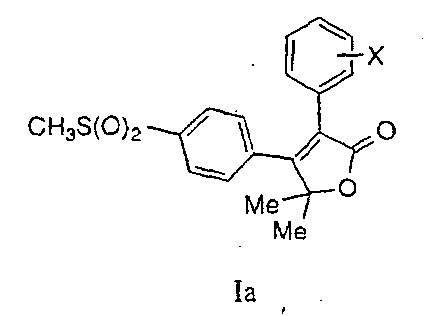 wobei X Fluor oder Chlor ist,
wobei das Verfahren umfaßt:
(a) Umsetzung von Thioanisol 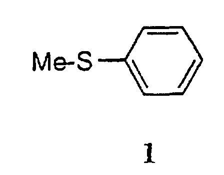 in einem nichtreaktiven Lösungsmittel und in Gegenwart einer Lewissäure mit Isobutyrylchlorid 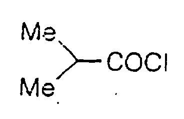 um Verbindung **2** zu ergeben: 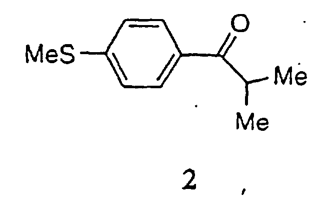
(b) Umsetzung einer Verbindung **2** mit einem Bromierungsmittel in einem nichtreaktiven Lösungsmittel in Gegenwart eines zweiten Lösungsmittels, um Verbindung **3** zu ergeben: 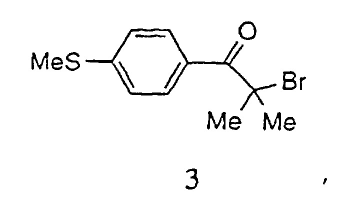
(c) Oxidation von Verbindung **3** in einem nichtreaktiven Lösungsmittel mit einem Oxidationsmittel, gegebenenfalls in Gegenwart eines geeigneten Katalysators, um Verbindung **4** zu ergeben: 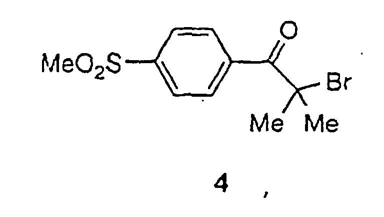
(d) Umsetzung von Verbindung **4** in einem Alkanol-Lösungsmittel mit Verbindung **5a** 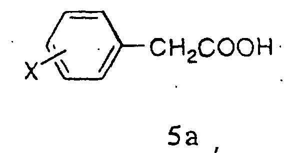
wobei X F oder Cl ist, in Gegenwart einer geeigneten Base, um eine Verbindung der Formel Ia zu ergeben

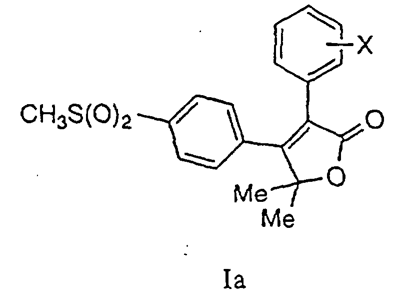

15. Ein Verfahren zur Herstellung von Verbindungen der Formel Ia: 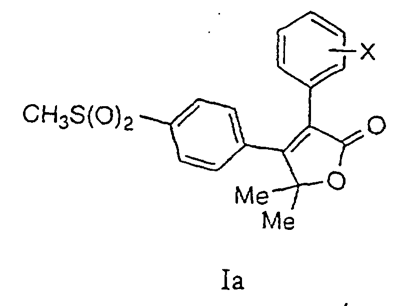 wobei X Fluor oder Chlor ist, umfassend:
(d) Umsetzung von Verbindung **4**: 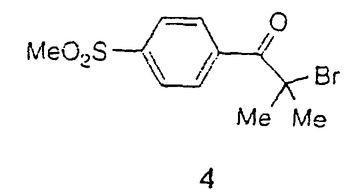 in einem Alkanol-Lösungsmittel mit Verbindung 5a 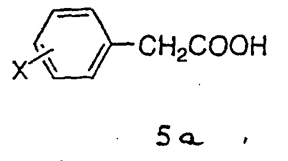 wobei X F oder Cl ist,
in Gegenwart einer geeigneten Base, um eine Verbindung der Formel Ia zu ergeben

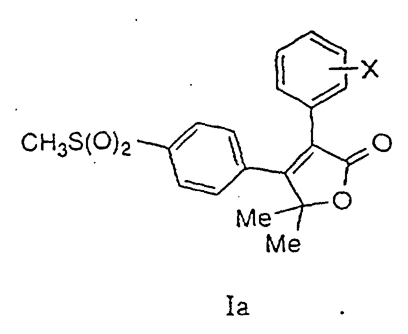

16. Ein Verfahren gemäß Anspruch 15 zur Herstellung von Verbindungen der Formel Ia 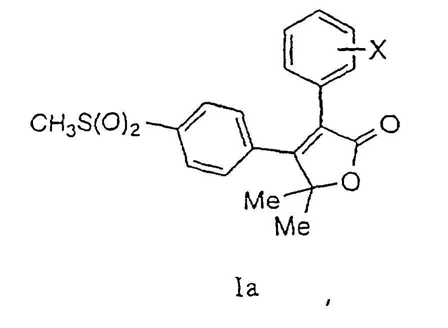 wobei X Fluor oder Chlor ist,
umfassend:
(c) Oxidation von Verbindung **3** 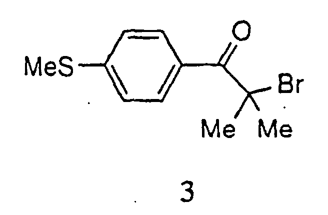 in einem nichtreaktiven Lösungsmittel mit einem Oxidationsmittel, gegebenenfalls in Gegenwart eines geeigneten Katalysators, um Verbindung **4** zu ergeben 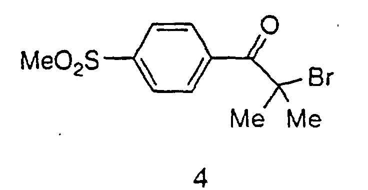
und (d) Umsetzung von Verbindung **4** in einem Alkanol-Lösungsmittel mit Verbindung **5a** 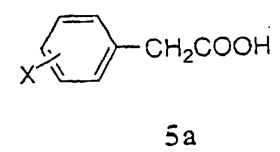 in Gegenwart einer geeigneten Base, um eine Verbindung der Formel Ia zu ergeben

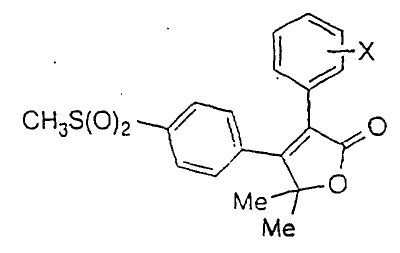

17. Ein Verfahren gemäß Anspruch 16 zur Herstellung von Verbindungen der Formel Ia: 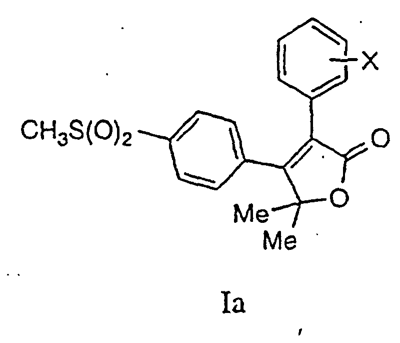 wobei X Fluor oder Chlor ist,
umfassend:
(b) Umsetzung von Verbindung 2: 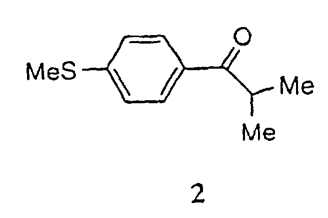 mit einem Bromierungsmittel in einem nichtreaktiven Lösungsmittel in Gegenwart eines zweiten Lösungsmittels, um Verbindung **3** zu ergeben: 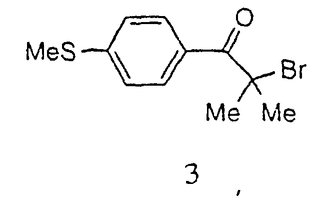
(c) Oxidation von Verbindung 3 in einem nichtreaktiven Lösungsmittel mit einem Oxidationsmittel, gegebenenfalls in Gegenwart eines geeigneten Katalysators, um Verbindung **4** zu ergeben: 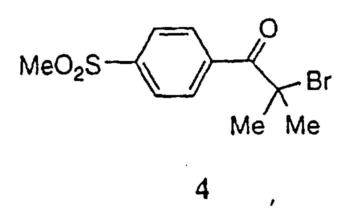
und (d) Umsetzung von Verbindung **4** in einem Alkanol-Lösungsmittel mit Verbindung **5a** 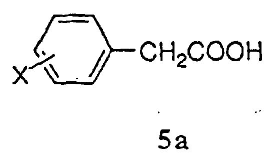 in Gegenwart einer geeigneten Base, um eine Verbindung der Formel Ia zu ergeben:

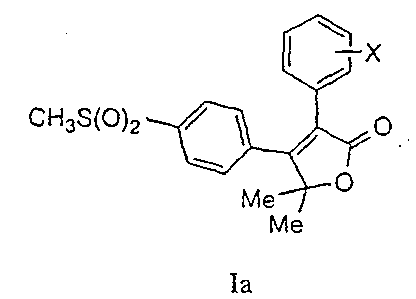

18. Ein Verfahren gemäß Anspruch 14, 15, 16 oder 17, wobei X 3-Fluor ist.

19. Ein Verfahren zur Herstellung von Verbindungen der Formel **4** oder **4a:** 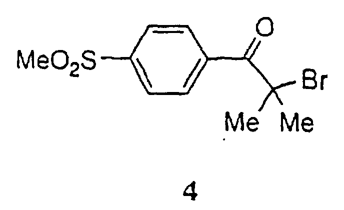 oder 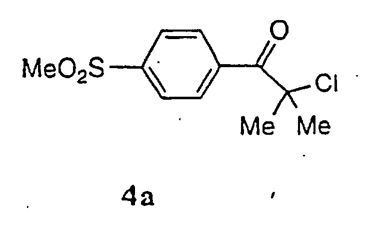 umfassend:
(a) Umsetzung von Thioanisol 1: 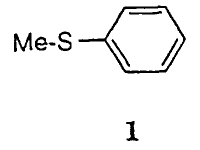 in einem nichtreaktiven Lösungsmittel und in Gegenwart einer Lewissäure mit Isobutyrylchlorid: 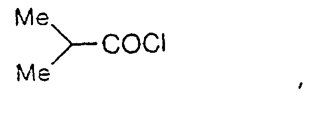 um Verbindung **2** zu ergeben 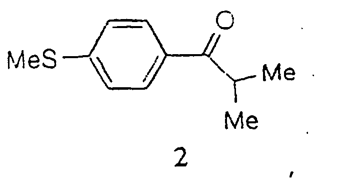
(b) Umsetzung von Verbindung **2** mit einem Bromierungsmittel oder mit einem Chlorierungsmittel in einem nichtreaktiven Lösungsmittel in Gegenwart eines zweiten Lösungsmittels, um eine Verbindung der Formel 3 oder **3a** zu ergeben: 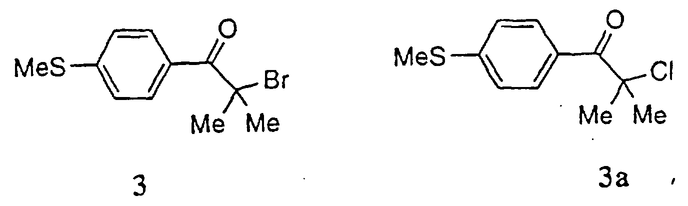 und (c) Oxidation von Verbindung **3** oder **3a** in einem nichtreaktiven Lösungsmittel (wie es oben definiert ist) mit einem Oxidationsmittel, gegebenenfalls in Gegenwart eines geeigneten Katalysators, um Verbindung **4** oder **4a** zu erbeben:

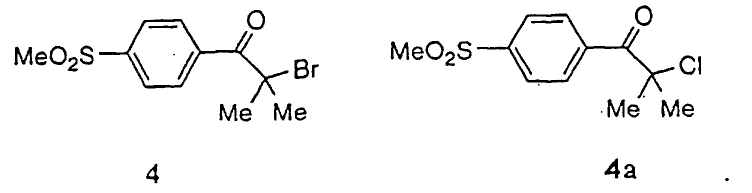

20. Ein Verfahren gemäß Anspruch 19, wobei das nichtreaktive Lösungsmittel Cyclohexan oder ortho-Dichlorbenzol ist.

21. Ein Verfahren gemäß Anspruch 19, wobei die Lewissäure Aluminiumchlorid ist.

22. Ein Verfahren gemäß Anspruch 19, wobei das Bromierungsmittel Brom ist.

23. Ein Verfahren gemäß Anspruch 19, wobei das Oxidationsmittel Wasserstoffperoxid ist.

24. Ein Verfahren gemäß Anspruch 19, wobei X 3-Fluor ist.

25. Eine Verbindung, die 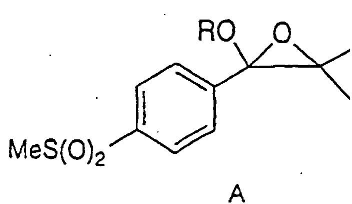 ist, wobei R C₁₋₆-Alkyl ist.

26. Eine Verbindung gemäß Anspruch 25, wobei R Ethyl ist.

## Revendications

1. Processus de fabrication des composés de formule I 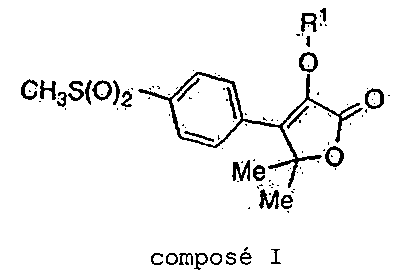
R¹ est choisi dans la catégorie constituée de :
(a) un alkyle C₁₋₆ linéaire ou ramifié,
(b) un phényle ou un naphthyle mono-, di- ou tri- substitué, dans lequel les substituants sont choisis dans la catégorie constituée de :
(1) un hydrogène,
(2) un halogène,
(3) un groupe alcoxy C₁₋₃,
(4) un groupe CN,
(5) un fluoroalkyle C₁₋₃,
(6) un alkyle C₁₋₃,
(7) un groupe -CO₂H,
le processus comprenant :
(a) faire réagir le thioanisole 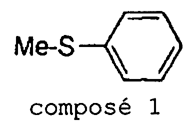 dans un solvant non-réactif et en présence d'un acide de Lewis, avec le chlorure d'isobutyryle. 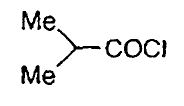 pour donner le composé 2 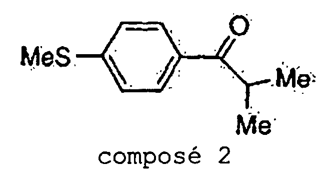
(b) faire subir une bromuration à un composé 2 avec un agent de bromuration dans un solvant non-réactif (comme défini ci-dessus), en présence d'un second solvant, tel que l'acétate d'éthyle pour donner le composé 3 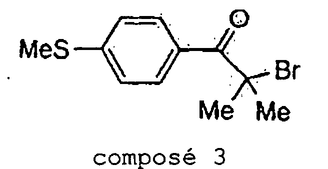
(c) oxyder le composé 3 dans un solvant non-réactif (comme défini ci-dessus), avec un agent oxydant, et de façon optionnelle, en présence d'un catalyseur adéquat pour donner le composé 4 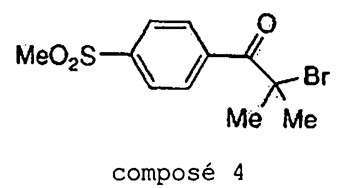
(d) faire réagir le composé 4 dans un solvant alcool avec le composé 5 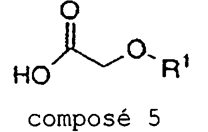 en présence d'une base adéquate pour donner un composé de formule 6 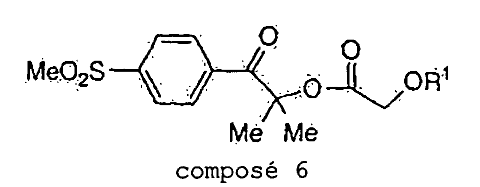
(e) faire réagir le composé 6 dans un solvant aprotique avec une base forte pour donner un composé cyclisé de formule 7. 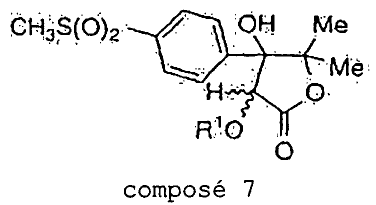 qui après déshydratation en présence d'un fixateur d'eau donne un composé de formule I.

2. Procédé de préparation du composé de formule I 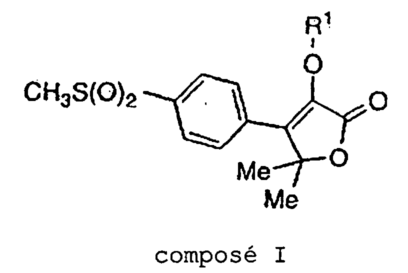
R¹ est choisi dans la catégorie constituée de :
(a) un alkyle C₁₋₆ linéaire ou ramifié,
(b) un phényle ou un naphthyle mono-, di- ou tri- substitué dans lequel les substituants sont choisis dans la catégorie constituée de :
(1) un hydrogène,
(2) un halogène,
(3) un groupe alcoxy C₁₋₃,
(4) un groupe CN,
(5) un fluoroalkyle C₁₋₃,
(6) un alkyle C₁₋₃,
(7) un groupe -CO₂H,
comprenant les étapes de:
(a) faire réagir le composé 6 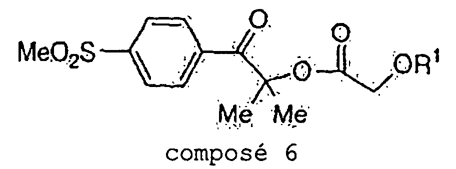 dans un solvant aprotique avec une base forte pour donner un composé cyclisé de formule 7. 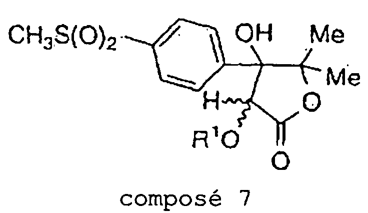 qui après déshydratation en présence d'un fixateur d'eau donne un composé de formule I

3. Procédé selon la revendication 2, dans lequel le fixateur d'eau est le trifluoroacétate d'isopropyle.

4. Procédé selon la revendication 2, dans lequel le solvant aprotique est l'acétonitrile.

5. Procédé selon la revendication 2, dans lequel la base forte est le 1,8-diazabicyclo[5,4,0]undec-7-ène.

6. Procédé selon la revendication 2, dans lequel le fixateur d'eau est le trifluoroacétate d'isopropyle, le solvant aprotique est l'acétonitrile et la base forte est le 1,8-diazabicyclo[5,4,0]undec-7-ène.

7. Procédé selon la revendication 6, dans lequel R¹ est l'isopropyle.

8. Procédé selon les revendications 2 ou 7, pour fabriquer des composés de formule I comprenant les étapes de :
(d) faire réagir le composé 4 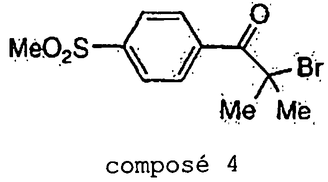 dans un solvant alcool avec le composé 5 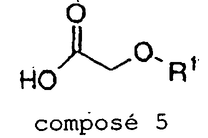 en présence d'une base adéquate pour donner un composé de formule 6 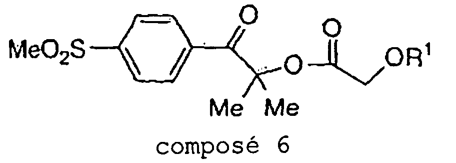
(e) faire réagir le composé 6 dans un solvant aprotique avec une base forte pour donner un composé cyclisé de formule 7. 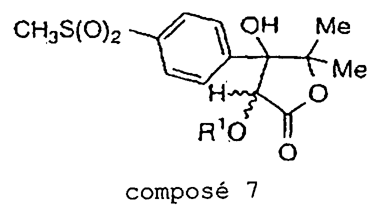 qui après déshydratation en présence d'un fixateur d'eau donne un composé de formule I.

9. Procédé selon la revendication 8, pour la fabrication de composés de formule I comprenant les étapes de :
(c) oxyder le composé 3 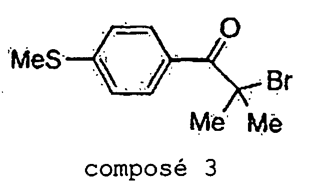 dans un solvant non-réactif (comme défini ci-dessus), avec un agent oxydant, et de façon optionnelle, en présence d'un catalyseur adéquat pour donner le composé 4 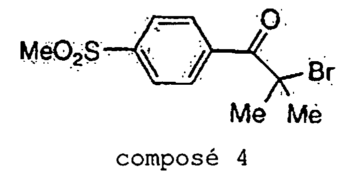
(d) faire réagir le composé 4 dans un solvant alcool avec le composé 5 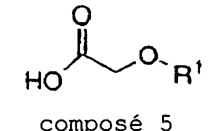 en présence d'une base adéquate pour donner un composé de formule 6 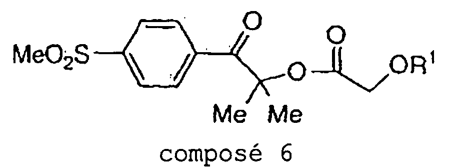
(e) faire réagir le composé 6 dans un solvant aprotique avec une base forte pour donner un composé cyclisé de formule 7. 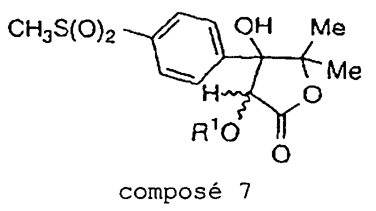 qui après déshydratation en présence d'un fixateur d'eau donne un composé de formule I.

10. Procédé selon la revendication 9 pour la fabrication d'un composé de formule I comprenant les étapes de :
(b) faire subir une bromuration à un composé 2 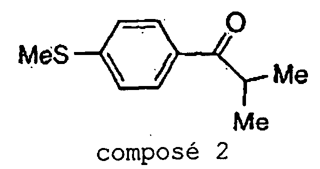 avec un agent de bromuration dans un solvant non-réactif, en présence d'un second solvant, tel que l'acétate d'éthyle pour donner le composé 3 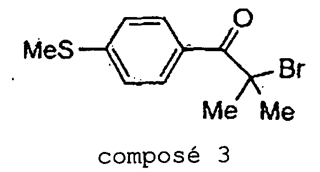
(c) oxyder le composé 3 dans un solvant non-réactif (comme défini ci-dessus), avec un agent oxydant, et de façon optionnelle, en présence d'un catalyseur adéquat pour donner le composé 4 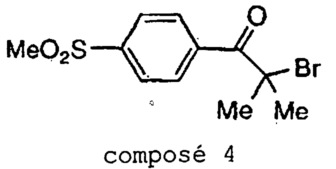
(d) faire réagir le composé 4 dans un solvant alcool avec le composé 5 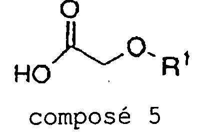 en présence d'une base adéquate pour donner un composé de formule 6 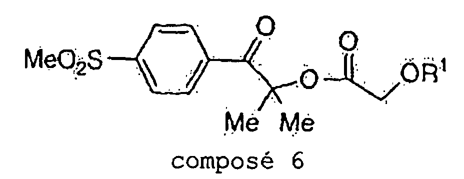
(e) faire réagir le composé 6 dans un solvant aprotique avec une base forte pour donner un composé cyclisé de formule 7. 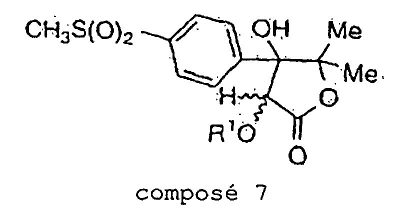 qui après déshydratation en présence d'un fixateur d'eau donne un composé de formule I.

11. Composé de formule : 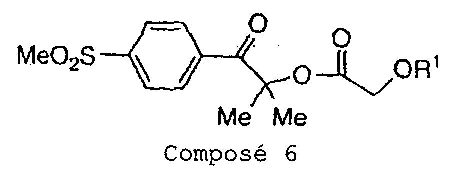 ou 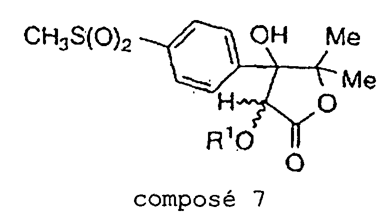 dans lequel
R¹ est choisi dans la catégorie constituée de :
(a) un alkyle C₁₋₆ linéaire ou ramifié,
(b) un phényle ou un naphthyle mono-, di- ou tri- substitué dans lequel les substituants sont choisis dans la catégorie constituée de :
(1) un hydrogène,
(2) un halogène,
(3) un groupe alcoxy C₁₋₃,
(4) un groupe CN,
(5) un fluoroalkyle C₁₋₃,
(6) un alkyle C₁₋₃,
(7) un groupe -CO₂H.

12. Composé selon la revendication 11, dans lequel R¹ est l'isopropyle.

13. Procédé de préparation de composés de formule Ia 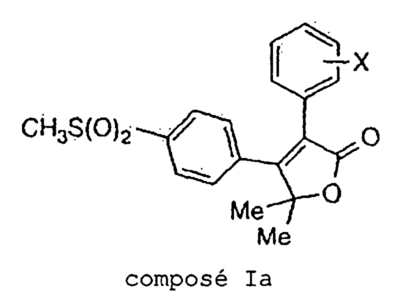
X est H, un fluor ou un chlore, dans lequel il y a 1, 2 ou 3 groupements X ; le processus comprenant :
(a) faire réagir le thioanisole 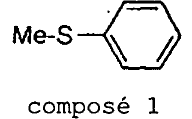 dans un solvant non-réactif et en présence d'un acide de Lewis, avec le chlorure d'isobutyryle. 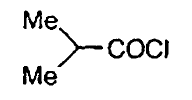 pour donner le composé 2 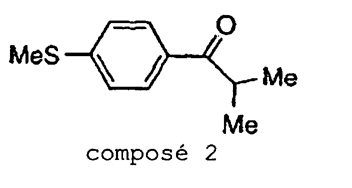
(b) faire subir une bromuration à un composé 2 avec un agent de bromuration dans un solvant non-réactif (comme défini ci-dessus), en présence d'un second solvant pour donner le composé 3 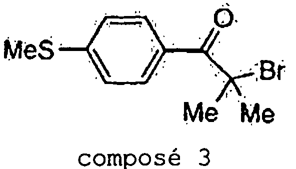 ou chlorer un composé 2 avec un agent de chloration dans un solvant non-réactif (comme défini ci-dessus), en présence d'un second solvant pour donner le composé 3a 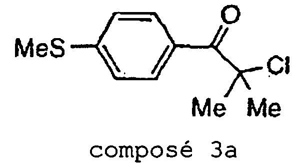
(c) oxyder le composé 3 ou 3a dans un solvant non-réactif (comme défini ci-dessus), avec un agent oxydant, et de façon optionnelle, en présence d'un catalyseur adéquat pour donner le composé 4 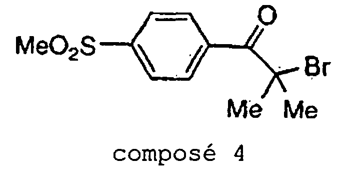 ou 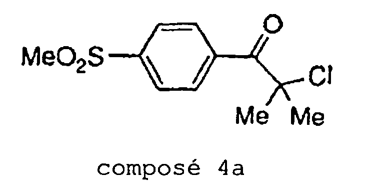
(d) faire réagir le composé 4 ou 4a dans un solvant alcool avec le composé 5a 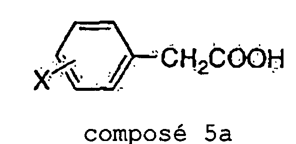 en présence d'une base adéquate pour donner un composé de formule Ia

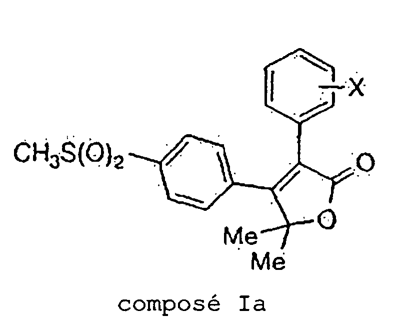

14. Procédé de préparation de composés de formule Ia 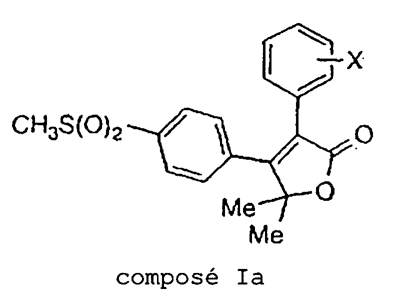
X est un fluor ou un chlore
Le processus comprenant :
(a) faire réagir le thioanisole 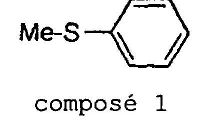 dans un solvant non-réactif et en présence d'un acide de Lewis, avec le chlorure d'isobutyryle. 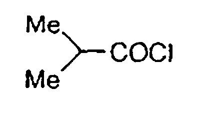 pour donner le composé 2 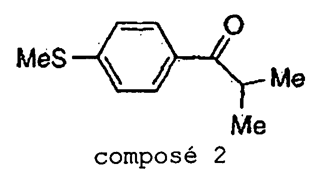
(b) faire réagir un composé 2 avec un agent de bromuration dans un solvant non-réactif, en présence d'un second solvant pour donner le composé 3 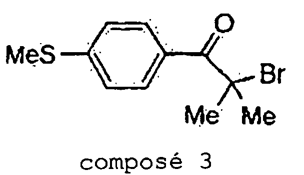
(c) oxyder le composé 3 dans un solvant non-réactif, avec un agent oxydant, et de façon optionnelle, en présence d'un catalyseur adéquat pour donner le composé 4 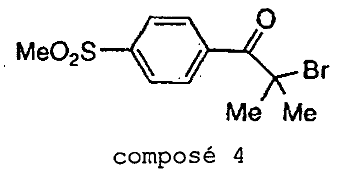
(d) faire réagir le composé 4 dans un solvant alcool avec le composé 5a 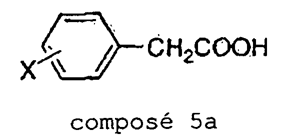
dans lequel X est F ou Cl ;
en présence d'une base adéquate pour donner un composé de formule Ia

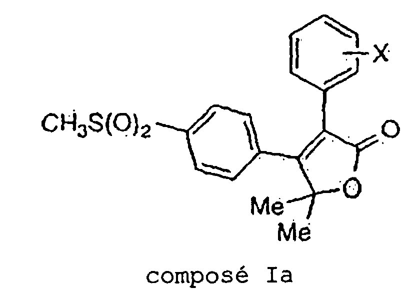

15. Procédé de préparation de composés de formule Ia : 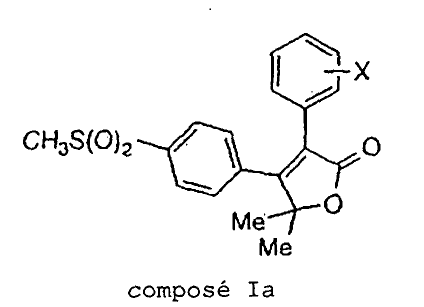 dans lequel X est un fluor ou un chlore ; comprenant :
(d) faire réagir le composé 4 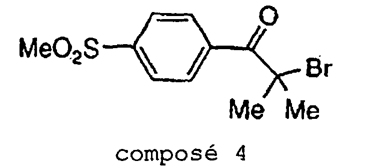 dans un solvant alcool avec le composé 5a 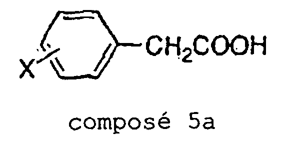 dans lequel X est F ou Cl ;
en présence d'une base adéquate pour donner un composé de formule Ia

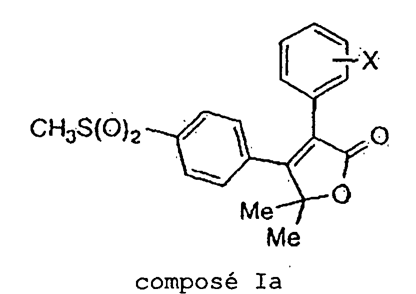

16. Procédé selon la revendication 15, pour la fabrication de composés de formules Ia 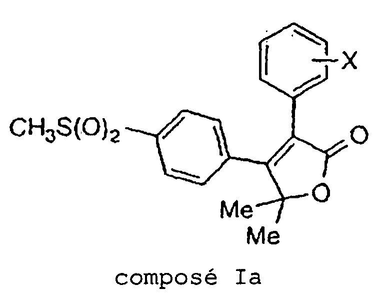 dans lequel X est un fluor ou un chlore ; comprenant :
(c) oxyder le composé 3 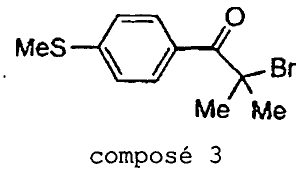 dans un solvant non-réactif, avec un agent oxydant, et de façon optionnelle, en présence d'un catalyseur adéquat pour donner le composé 4 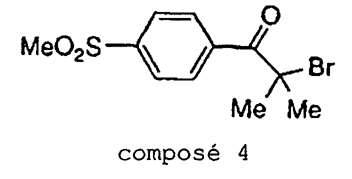 et
(d) faire réagir le composé 4 dans un solvant alcool avec le composé 5a 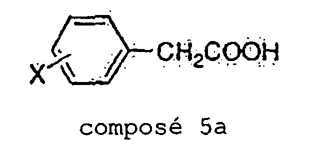 en présence d'une base adéquate pour donner un composé de formule Ia

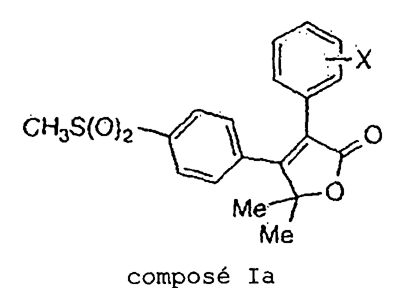

17. Procédé selon la revendication 16, pour la fabrication de composés de formule Ia : 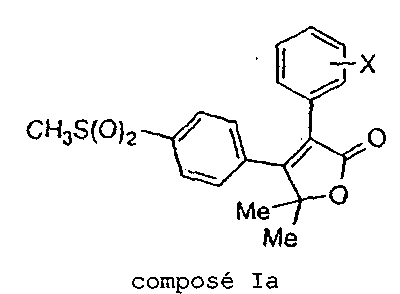 dans lequel X est un fluor ou un chlore ; comprenant :
(b) faire réagir un composé 2 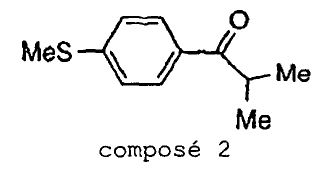 avec un agent de bromuration dans un solvant non-réactif, en présence d'un second solvant, pour donner le composé 3 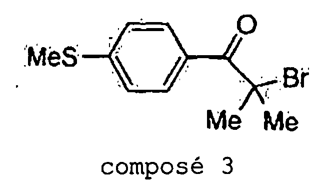
(c) oxyder le composé 3 dans un solvant non-réactif, avec un agent oxydant, et de façon optionnelle, en présence d'un catalyseur adéquat pour donner le composé 4 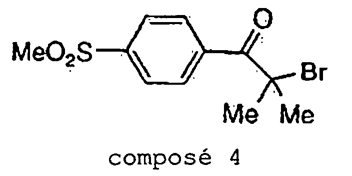 et
(d) faire réagir le composé 4 dans un solvant alcool avec le composé 5a 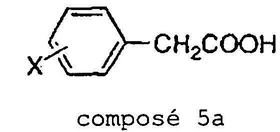 en présence d'une base adéquate pour donner un composé de formule Ia

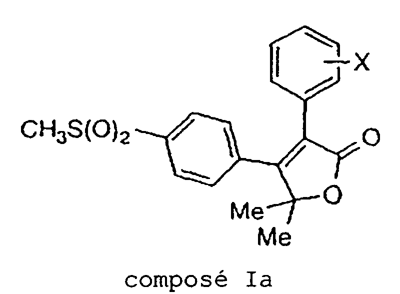

18. Procédé selon les revendications 14, 15, 16 ou 17, dans lequel X est le groupement 3-fluoro.

19. Procédé de préparation de composés de formule 4 ou 4a : 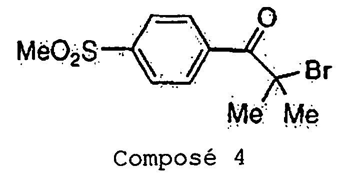 ou 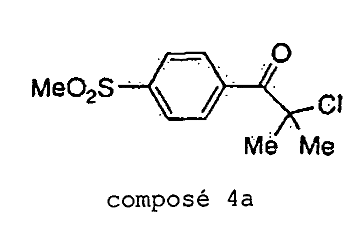 comprenant :
(a) faire réagir le thioanisole 1 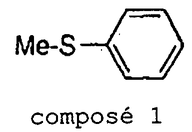 dans un solvant non-réactif et en présence d'un acide de Lewis, avec le chlorure d'isobutyryle. 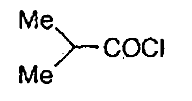 pour donner le composé 2 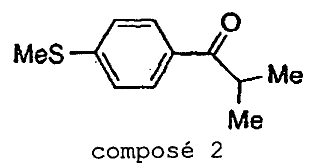
(b) faire réagir un composé 2 avec un agent de bromuration ou un agent de chloration dans un solvant non-réactif, en présence d'un second solvant, pour donner le composé 3 ou 3a : 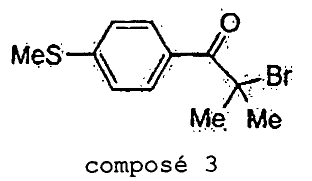 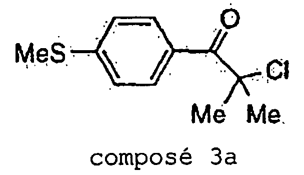 et
(c) oxyder le composé 3 ou 3a dans un solvant non-réactif (comme défini ci-dessus), avec un agent oxydant, et de façon optionnelle, en présence d'un catalyseur adéquat pour donner le composé 4 ou 4a :

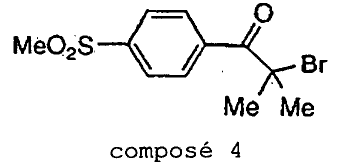

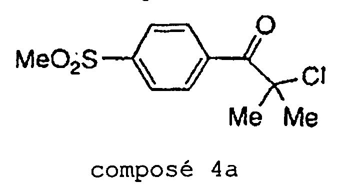

20. Procédé selon la revendication 19, dans lequel le solvant non-réactif est le cyclohexane ou l'orthodichlorobenzène.

21. Procédé selon la revendication 19, dans lequel l'acide de Lewis est le chlorure d'aluminium.

22. Procédé selon la revendication 19, dans lequel l'agent de bromuration est le brome.

23. Procédé selon la revendication 19, dans lequel l'agent oxydant est le peroxyde d'hydrogène.

24. Procédé selon la revendication 19, dans lequel X est un groupement 3-fluoro.

25. Composé qui est 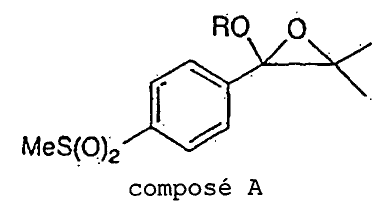 Dans lequel R est un alkyle C₁₋₆.

26. Composé selon la revendication 25, dans lequel R est l'éthyle.
